# EUROPEAN PATENT APPLICATION

(11) **EP 1 870 469 A1**
(43) Date of publication of application: **26.12.2007**
(21) Application number: 05793125.5
(22) Date of filing: 11.10.2005
(51) Int. Cl.: C12N 15/60, A01H 5/00, C12N 5/10, C12N 9/88, C12Q 1/02

(54) **NOVEL ALTERED GENE FROM RICE ANTHRANILIC ACID SYNTHASE GENE OASA2 AND USE THEREOF**

(30) Priority: 28.02.2005 JP 2005055165
(71) Applicant: NATIONAL UNIVERSITY CORPORATION EHIME UNIVERSITY, Matsuyama Ehime 790-08577 (JP); Japan Science and Technology Agency, Kawaguchi-shi, Saitama 332-0012 (JP); Incorporated Administrative Agency National Agriculture and Food Research Organization, Ibaraki 305-8517 (JP)
(72) Inventor: TOZAWA, Yuzuru, Matsuyama-shi, Ehime 790-0855 (JP); KANNO, Takuya, Isehara-shi, Kanagawa 259-1114 (JP); WAKASA, Kyo, Nat. Agriculture and Food Res.Org., Tsukuba-shi, Ibaraki 305-8518 (JP)
(74) Representative: Bittner, Thomas L.
(86) International application number: PCT/JP2005/018708
(87) International publication number: WO 2006/092882

(57) **Abstract**

A rice anthranilic acid synthase produced by introducing a variation in the base sequence of rice anthranilic acid synthase gene OASA2 so as to effect substitution for specified multiple amino acids. This synthase not only acquires a resistance to feedback inhibition by tryptophan but also retains an enzymatic activity identical with or higher than that of wild type rice anthranilic acid synthase.

SEQUENCE LISTING

<110> National University Corporation Ehime University
      Japan Science and Technology Agency
      Incorporated Administrative Agency National Agriculture and Food Research
Organization

<120> Novel altered gene from rice anthranilic acid synthase gene OASA2 and use
thereof

<130> A181-14

<150> JP 2005-055165
<151> 2005-02-28

<160> 46

<170> PatentIn Ver. 2.1

<210> 1
<211> 606
<212> PRT
<213> Oryza sativa

<400> 1
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser
     50                  55                  60

Ala Ala Ala Arg Ala Ala Glu Glu Asp Arg Arg Arg Phe Phe Glu Ala
 65                  70                  75                  80

Ala Glu Arg Gly Ser Gly Lys Gly Asn Leu Val Pro Met Trp Glu Cys
                 85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Ser Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly
    210                 215                 220

Ala Pro Gln Asp Asp Arg Asn Leu Pro Asp Val His Leu Gly Leu Tyr
225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asn Pro
            340                 345                 350

Phe Glu Val Tyr Arg Ala Leu Arg Ile Val Asn Pro Ser Pro Tyr Met
        355                 360                 365

Ala Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala
385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Gly Ile Ser Phe Asp Gly Asp
        515                 520                 525

Met Leu Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu
545                 550                 555                 560

Trp Val Ala His Leu Gln Ala Gly Ala Gly Ile Val Ala Asp Ser Ser
                565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605

<210> 2
<211> 606
<212> PRT
<213> Artificial Sequence

<220>
<223> Description of Artificial Sequence:Modified amino
      acid sequence of Sequence No.1

<400> 2
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser
     50                  55                  60

Ala Ala Ala Arg Ala Ala Glu Glu Asp Arg Arg Arg Phe Phe Glu Ala
 65                  70                  75                  80

Ala Glu Arg Gly Ser Gly Lys Gly Asn Leu Val Pro Met Trp Glu Cys
                 85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Ser Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly
    210                 215                 220

Ala Pro Gln Asp Asp Arg Asn Leu Pro Asp Val His Leu Gly Leu Tyr
225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asn Pro
            340                 345                 350

Phe Glu Val Tyr Arg Ala Leu Arg Ile Val Asn Pro Ser Pro Ala Met
        355                 360                 365

Ala Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala
385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Gly Ile Ser Phe Asp Gly Asp
        515                 520                 525

Met Leu Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu
545                 550                 555                 560

Trp Val Ala His Leu Gln Ala Gly Ala Gly Ile Val Ala Asp Ser Ser
                565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605


<210> 3
<211> 606
<212> PRT
<213> Artificial Sequence

<220>
<223> Description of Artificial Sequence:Modified amino
      acid sequence of Sequence No.1

<400> 3
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser
     50                  55                  60

Ala Ala Ala Arg Ala Ala Glu Glu Asp Arg Arg Arg Phe Phe Glu Ala
 65                  70                  75                  80

Ala Glu Arg Gly Ser Gly Lys Gly Asn Leu Val Pro Met Trp Glu Cys
                 85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Ser Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly

210                 215                 220

Ala Pro Gln Asp Asp Arg Asn Leu Pro Asp Val His Leu Gly Leu Tyr
225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asn Pro
            340                 345                 350

Phe Glu Val Tyr Arg Ala Leu Arg Ile Val Asn Pro Ser Pro Ile Met
        355                 360                 365

Ala Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala
385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Gly Ile Ser Phe Asp Gly Asp
        515                 520                 525

Met Leu Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu

545                 550                 555                 560

Trp Val Ala His Leu Gln Ala Gly Ala Gly Ile Val Ala Asp Ser Ser
                565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605


<210> 4
<211> 606
<212> PRT
<213> Artificial Sequence

<220>
<223> Description of Artificial Sequence:Modified amino
      acid sequence of Sequence No.1

<400> 4
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser
     50                  55                  60

Ala Ala Ala Arg Ala Ala Glu Glu Asp Arg Arg Arg Phe Phe Glu Ala
 65                  70                  75                  80

Ala Glu Arg Gly Ser Gly Lys Gly Asn Leu Val Pro Met Trp Glu Cys
                 85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Ser Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly
    210                 215                 220

Ala Pro Gln Asp Asp Arg Asn Leu Pro Asp Val His Leu Gly Leu Tyr
225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asn Pro
            340                 345                 350

Phe Glu Val Tyr Arg Ala Leu Arg Ile Val Asn Pro Ser Pro Ala Met
        355                 360                 365

Ala Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala
385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Gly Ile Ser Phe Asp Gly Asp
        515                 520                 525

Met Asp Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu
545                 550                 555                 560

Trp Val Ala His Leu Gln Ala Gly Ala Gly Ile Val Ala Asp Ser Ser
                565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605


<210> 5
<211> 606
<212> PRT
<213> Artificial Sequence

<220>
<223> Description of Artificial Sequence:Modified amino
      acid sequence of Sequence No.1

<400> 5
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser
     50                  55                  60

Ala Ala Ala Arg Ala Ala Glu Glu Asp Arg Arg Arg Phe Phe Glu Ala
 65                  70                  75                  80

Ala Glu Arg Gly Ser Gly Lys Gly Asn Leu Val Pro Met Trp Glu Cys
                 85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Ser Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly
    210                 215                 220

Ala Pro Gln Asp Asp Arg Asn Leu Pro Asp Val His Leu Gly Leu Tyr
225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asp Pro
            340                 345                 350

Phe Glu Val Tyr Arg Ala Leu Arg Ile Val Asn Pro Ser Pro Ala Met
        355                 360                 365

Ala Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala
385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Gly Ile Ser Phe Asp Gly Asp
        515                 520                 525

Met Asp Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu
545                 550                 555                 560

Trp Val Ala His Leu Gln Ala Gly Ala Gly Ile Val Ala Asp Ser Ser
                565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605

<210> 6
<211> 606
<212> PRT
<213> Artificial Sequence

<220>
<223> Description of Artificial Sequence:Modified amino
      acid sequence of Sequence No.1

<400> 6
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser
     50                  55                  60

Ala Ala Ala Arg Ala Ala Glu Glu Asp Arg Arg Arg Phe Phe Glu Ala
 65                  70                  75                  80

Ala Glu Arg Gly Ser Gly Lys Gly Asn Leu Val Pro Met Trp Glu Cys
                 85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Ser Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly
    210                 215                 220

Ala Pro Gln Asp Asp Arg Asn Leu Pro Asp Val His Leu Gly Leu Tyr

225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asn Pro
            340                 345                 350

Phe Glu Val Tyr Arg Ala Leu Arg Ile Val Asn Pro Ser Pro Ala Met
        355                 360                 365

Ala Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala
385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Tyr Ile Ser Phe Asp Gly Asp
        515                 520                 525

Met Asp Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu
545                 550                 555                 560

Trp Val Ala His Leu Gln Ala Gly Ala Gly Ile Val Ala Asp Ser Ser

565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605

<210> 7
<211> 606
<212> PRT
<213> Artificial Sequence

<220>
<223> Description of Artificial Sequence:Modified amino
      acid sequence of Sequence No.1

<400> 7
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser
     50                  55                  60

Ala Ala Ala Arg Ala Ala Glu Glu Asp Arg Arg Arg Phe Phe Glu Ala
 65                  70                  75                  80

Ala Glu Arg Gly Ser Gly Lys Gly Asn Leu Val Pro Met Trp Glu Cys
                 85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Ser Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly
    210                 215                 220

Ala Pro Gln Asp Asp Arg Asn Leu Pro Asp Val His Leu Gly Leu Tyr
225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asn Pro
            340                 345                 350

Phe Glu Val Tyr Arg Ala Leu Arg Ile Val Asn Pro Ser Pro Ile Met
        355                 360                 365

Ala Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala
385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Tyr Ile Ser Phe Asp Gly Asp
        515                 520                 525

Met Asp Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu
545                 550                 555                 560

Trp Val Ala His Leu Gln Ala Gly Ala Gly Ile Val Ala Asp Ser Ser
                565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605

<210> 8
<211> 1821
<212> DNA
<213> Oryza sativa

<400> 8
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180
atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360
agcttcctct tcgagtccgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600
ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780
cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacatacgcc aatccatttg aagtctatcg agctttacga 1080
attgtgaacc caagtccata catggcatat gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380
tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560
ggagggatat catttgacgg agacatgctt atcgctcttg cactccgcac cattgtgttc 1620
tcaacagcgc caagccacaa cacgatgtac tcatacaaag acaccgagag gcgccgggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g                                           1821

<210> 9
<211> 1821
<212> DNA
<213> Artificial Sequence

<220>
<223> Description of Artificial Sequence:Modified DNA
      sequence of Sequence No.8

<400> 9
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180
atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360

agcttcctct tcgagtccgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600
ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780
cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacatacgcc aatccatttg aagtctatcg agctttacga 1080
attgtgaacc caagtccagc catggcatac gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380
tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560
ggagggatat catttgacgg agacatgctt atcgctcttg cactccgcac cattgtgttc 1620
tcaacagcgc caagccacaa cacgatgtac tcatacaaag acaccgagag gcgccgggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g 1821

<210> 10
<211> 1821
<212> DNA
<213> Artificial Sequence

<220>
<223> Description of Artificial Sequence:Modified DNA
      sequence of Sequence No.8

<400> 10
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180
atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360
agcttcctct tcgagtccgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600
ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780
cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacatacgcc aatccatttg aagtctatcg agctttacga 1080
attgtgaacc caagtccaat catggcatac gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380
tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560

ggagggatat catttgacgg agacatgctt atcgctcttg cactccgcac cattgtgttc 1620
tcaacagcgc caagccacaa cacgatgtac tcatacaaag acaccgagag gcgccgggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g 1821

<210> 11
<211> 1821
<212> DNA
<213> Artificial Sequence

<220>
<223> Description of Artificial Sequence:Modified DNA
      sequence of Sequence No.8

<400> 11
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180
atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360
agcttcctct tcgagtccgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600
ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780
cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacatacgcc aatccatttg aagtctatcg agctttacga 1080
attgtgaacc caagtccagc catggcatac gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380
tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560
ggagggatat catttgacgg agacatggac atcgcgctag cactccgcac cattgtgttc 1620
tcaacagcgc caagccacaa cacgatgtac tcatacaaag acaccgagag gcgccgggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g 1821

<210> 12
<211> 1821
<212> DNA
<213> Artificial Sequence

<220>
<223> Description of Artificial Sequence:Modified DNA
      sequence of Sequence No.8

<400> 12
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180

atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360
agcttcctct tcgagtccgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600
ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780
cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacgtacgcc gatccatttg aagtctatcg agctttacga 1080
attgtgaacc caagtccagc catggcatac gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380
tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560
ggagggatat catttgacgg agacatggac atcgcgctag cactccgcac cattgtgttc 1620
tcaacagcgc caagccacaa cacgatgtac tcatacaaag acaccgagag gcgccgggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g 1821

<210> 13
<211> 1821
<212> DNA
<213> Artificial Sequence

<220>
<223> Description of Artificial Sequence:Modified DNA
      sequence of Sequence No.8

<400> 13
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180
atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360
agcttcctct tcgagtccgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600
ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780
cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacatacgcc aatccatttg aagtctatcg agctttacga 1080
attgtgaacc caagtccagc catggcatac gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380

tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560
ggatacatat catttgacgg agacatggac atcgcgctag cactccgcac cattgtgttc 1620
tcaacagcgc caagccacaa cacgatgtac tcatacaaag acaccgagag gcgccgggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g 1821

<210> 14
<211> 1821
<212> DNA
<213> Artificial Sequence

<220>
<223> Description of Artificial Sequence:Modified DNA
      sequence of Sequence No.8

<400> 14
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180
atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360
agcttcctct tcgagtccgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600
ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780
cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacatacgcc aatccatttg aagtctatcg agctttacga 1080
attgtgaacc caagtccaat catggcatac gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380
tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560
ggatacatat catttgacgg agacatggac atcgcgctag cactccgcac cattgtgttc 1620
tcaacagcgc caagccacaa cacgatgtac tcatacaaag acaccgagag gcgccgggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g 1821

<210> 15
<211> 38
<212> DNA
<213> Artificial Sequence

<220>
<223> Description of Artificial Sequence:Primer for
      site-directed mutagenesis

<400> 15

gtttgagagg cgaacgtacg ccgatccatt tgaagtct 38

<210> 16
<211> 42
<212> DNA
<213> Artificial Sequence

<220>
<223> Description of Artificial Sequence:Primer for
      site-directed mutagenesis

<400> 16
gtgaacccaa gtccagccat ggcatacgta caggcaagag gc 42

<210> 17
<211> 42
<212> DNA
<213> Artificial Sequence

<220>
<223> Description of Artificial Sequence:Primer for
      site-directed mutagenesis

<400> 17
gtgaacccaa gtccaatcat ggcatacgta caggcaagag gc 42

<210> 18
<211> 38
<212> DNA
<213> Artificial Sequence

<220>
<223> Description of Artificial Sequence:Primer for
      site-directed mutagenesis

<400> 18
acggagacat ggctatcgcg ctagcactcc gcaccatt 38

<210> 19
<211> 38
<212> DNA
<213> Artificial Sequence

<220>
<223> Description of Artificial Sequence:Primer for
      site-directed mutagenesis

<400> 19
acggagacat ggacatcgcg ctagcactcc gcaccatt 38

<210> 20
<211> 56
<212> DNA
<213> Artificial Sequence

<220>
<223> Description of Artificial Sequence:Primer for
      site-directed mutagenesis

<400> 20
agtggcggcc ttggatacat atcatttgac ggagacatgg atatcgctct tgcact     56

<210> 21
<211> 36
<212> DNA
<213> Artificial Sequence

<220>
<223> Description of Artificial Sequence:Artificially
      synthesized primer sequence

<400> 21
cctcttccag ggcccaatgt gctccgcggg gaagcc                           36

<210> 22
<211> 20
<212> DNA
<213> Artificial Sequence

<220>
<223> Description of Artificial Sequence:Artificially
      synthesized primer sequence

<400> 22
ggagaaaggc ggacaggtat                                             20

<210> 23
<211> 20
<212> DNA
<213> Artificial Sequence

<220>
<223> Description of Artificial Sequence:Artificially
      synthesized primer sequence

<400> 23
ggggaaacgc ctggtatctt                                             20

<210> 24
<211> 21
<212> DNA
<213> Artificial Sequence

<220>
<223> Description of Artificial Sequence:Artificially
      synthesized primer sequence

<400> 24
gcgtagcatt taggtgacac t                                           21

<210> 25
<211> 103
<212> DNA
<213> Artificial Sequence

<220>

<223> Description of Artificial Sequence:Artificially
      synthesized primer sequence

<400> 25
ggtgacacta tagaagtatt tttacaacaa ttaccaacaa caacaacaaa caacaacaac 60
attacatttt acattctaca actacctctt ccagggccca atg                   103


<210> 26
<211> 6
<212> PRT
<213> Oryza sativa

<400> 26
Asn Pro Ser Pro Tyr Met
  1               5


<210> 27
<211> 29
<212> DNA
<213> Artificial Sequence

<220>
<223> Description of Artificial Sequence:Artificially
      synthesized primer sequence

<400> 27
aaaggtacca tgtgctccgc ggggaagcc                                   29


<210> 28
<211> 32
<212> DNA
<213> Artificial Sequence

<220>
<223> Description of Artificial Sequence:Artificially
      synthesized primer sequence

<400> 28
aaagaattct gagagagact ctattccttg tc                               32


<210> 29
<211> 606
<212> PRT
<213> Artificial Sequence

<220>
<223> Description of Artificial Sequence:Modified amino
      acid sequence of Sequence No.1

<400> 29
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser
50                  55                  60

Ala Ala Ala Arg Ala Ala Glu Glu Asp Arg Arg Arg Phe Phe Glu Ala
65                  70                  75                  80

Ala Glu Arg Gly Ser Gly Lys Gly Asn Leu Val Pro Met Trp Glu Cys
                85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Phe Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly
    210                 215                 220

Ala Pro Gln Asp Asp Arg Asn Leu Pro Asp Val His Leu Gly Leu Tyr
225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asn Pro
            340                 345                 350

Phe Glu Val Tyr Arg Ala Leu Arg Ile Val Asn Pro Ser Pro Tyr Met
        355                 360                 365

Ala Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala
385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Gly Ile Ser Phe Asp Gly Asp
        515                 520                 525

Met Leu Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu
545                 550                 555                 560

Trp Val Ala His Leu Gln Ala Gly Ala Gly Ile Val Ala Asp Ser Ser
                565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605


<210> 30
<211> 606
<212> PRT
<213> Artificial Sequence

<220>
<223> Description of Artificial Sequence:Modified amino
      acid sequence of Sequence No.1

<400> 30
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser

50                  55                  60

Ala Ala Ala Arg Ala Ala Glu Glu Asp Arg Arg Arg Phe Phe Glu Ala
65                  70                  75                  80

Ala Glu Arg Gly Ser Gly Lys Gly Asn Leu Val Pro Met Trp Glu Cys
                85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Ser Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly
    210                 215                 220

Ala Pro Gln Asp Asp Arg Asn Leu Pro Asp Val His Leu Gly Leu Tyr
225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asn Pro
            340                 345                 350

Phe Glu Val Tyr Arg Ala Leu Arg Ile Val Asn Pro Ser Pro Tyr Met
        355                 360                 365

Leu Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala

385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Gly Ile Ser Phe Asp Gly Asp
        515                 520                 525

Met Leu Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu
545                 550                 555                 560

Trp Val Ala His Leu Gln Ala Gly Ala Gly Ile Val Ala Asp Ser Ser
                565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605


<210> 31
<211> 606
<212> PRT
<213> Artificial Sequence

<220>
<223> Description of Artificial Sequence:Modified amino
      acid sequence of Sequence No.1

<400> 31
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser
     50                  55                  60

Ala Ala Ala Arg Ala Ala Glu Glu Asp Arg Arg Arg Phe Phe Glu Ala
65                  70                  75                  80

Ala Glu Arg Gly Ser Gly Lys Gly Asn Leu Val Pro Met Trp Glu Cys
                85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Phe Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly
    210                 215                 220

Ala Pro Gln Asp Asp Arg Asn Leu Pro Asp Val His Leu Gly Leu Tyr
225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asn Pro
            340                 345                 350

Phe Glu Val Tyr Arg Ala Leu Arg Ile Val Asn Pro Ser Pro Tyr Met
        355                 360                 365

Ala Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala
385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Gly Ile Ser Phe Asp Gly Asp
        515                 520                 525

Met Asp Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu
545                 550                 555                 560

Trp Val Ala His Leu Gln Ala Gly Ala Gly Ile Val Ala Asp Ser Ser
                565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605


<210> 32
<211> 606
<212> PRT
<213> Artificial Sequence

<220>
<223> Description of Artificial Sequence:Modified amino
      acid sequence of Sequence No.1

<400> 32
Met Glu Ser Ile Ala Ala Ala Thr Phe Thr Pro Ser Arg Leu Ala Ala
  1               5                  10                  15

Arg Pro Ala Thr Pro Ala Ala Ala Ala Ala Pro Val Arg Ala Arg Ala
             20                  25                  30

Ala Val Ala Ala Gly Gly Arg Arg Arg Thr Ser Arg Arg Gly Gly Val
         35                  40                  45

Arg Cys Ser Ala Gly Lys Pro Glu Ala Ser Ala Val Ile Asn Gly Ser
     50                  55                  60

Ala Ala Ala Arg Ala Ala Glu Glu Asp Arg Arg Arg Phe Phe Glu Ala
65                  70                  75                  80

Ala Glu Arg Gly Ser Gly Lys Gly Asn Leu Val Pro Met Trp Glu Cys
                85                  90                  95

Ile Val Ser Asp His Leu Thr Pro Val Leu Ala Tyr Arg Cys Leu Val
            100                 105                 110

Pro Glu Asp Asn Met Glu Thr Pro Ser Phe Leu Phe Glu Ser Val Glu
        115                 120                 125

Gln Gly Pro Glu Gly Thr Thr Asn Val Gly Arg Tyr Ser Met Val Gly
    130                 135                 140

Ala His Pro Val Met Glu Val Val Ala Lys Glu His Lys Val Thr Ile
145                 150                 155                 160

Met Asp His Glu Lys Gly Lys Val Thr Glu Gln Val Val Asp Asp Pro
                165                 170                 175

Met Gln Ile Pro Arg Ser Met Met Glu Gly Trp His Pro Gln Gln Ile
            180                 185                 190

Asp Gln Leu Pro Asp Ser Phe Thr Gly Gly Trp Val Gly Phe Phe Ser
        195                 200                 205

Tyr Asp Thr Val Arg Tyr Val Glu Lys Lys Lys Leu Pro Phe Ser Gly
    210                 215                 220

Ala Pro Gln Asp Asp Arg Asn Leu Pro Asp Val His Leu Gly Leu Tyr
225                 230                 235                 240

Asp Asp Val Leu Val Phe Asp Asn Val Glu Lys Lys Val Tyr Val Ile
                245                 250                 255

His Trp Val Asn Leu Asp Arg His Ala Thr Thr Glu Asp Ala Phe Gln
            260                 265                 270

Asp Gly Lys Ser Arg Leu Asn Leu Leu Leu Ser Lys Val His Asn Ser
        275                 280                 285

Asn Val Pro Lys Leu Ser Pro Gly Phe Val Lys Leu His Thr Arg Gln
    290                 295                 300

Phe Gly Thr Pro Leu Asn Lys Ser Thr Met Thr Ser Asp Glu Tyr Lys
305                 310                 315                 320

Asn Ala Val Met Gln Ala Lys Glu His Ile Met Ala Gly Asp Ile Phe
                325                 330                 335

Gln Ile Val Leu Ser Gln Arg Phe Glu Arg Arg Thr Tyr Ala Asn Pro
            340                 345                 350

Phe Glu Val Tyr Arg Ala Leu Arg Ile Val Asn Pro Ser Pro Tyr Met
        355                 360                 365

Leu Tyr Val Gln Ala Arg Gly Cys Val Leu Val Ala Ser Ser Pro Glu
    370                 375                 380

Ile Leu Thr Arg Val Arg Lys Gly Lys Ile Ile Asn Arg Pro Leu Ala
385                 390                 395                 400

Gly Thr Val Arg Arg Gly Lys Thr Glu Lys Glu Asp Glu Met Gln Glu
                405                 410                 415

Gln Gln Leu Leu Ser Asp Glu Lys Gln Cys Ala Glu His Ile Met Leu
            420                 425                 430

Val Asp Leu Gly Arg Asn Asp Val Gly Lys Val Ser Lys Pro Gly Ser
        435                 440                 445

Val Lys Val Glu Lys Leu Met Asn Ile Glu Arg Tyr Ser His Val Met
    450                 455                 460

His Ile Ser Ser Thr Val Ser Gly Glu Leu Asp Asp His Leu Gln Ser
465                 470                 475                 480

Trp Asp Ala Leu Arg Ala Ala Leu Pro Val Gly Thr Val Ser Gly Ala
                485                 490                 495

Pro Lys Val Lys Ala Met Glu Leu Ile Asp Glu Leu Glu Val Thr Arg
            500                 505                 510

Arg Gly Pro Tyr Ser Gly Gly Leu Gly Gly Ile Ser Phe Asp Gly Asp
        515                 520                 525

Met Asp Ile Ala Leu Ala Leu Arg Thr Ile Val Phe Ser Thr Ala Pro
    530                 535                 540

Ser His Asn Thr Met Tyr Ser Tyr Lys Asp Thr Glu Arg Arg Arg Glu
545                 550                 555                 560

Trp Val Ala His Leu Gln Ala Gly Ala Gly Ile Val Ala Asp Ser Ser
                565                 570                 575

Pro Asp Asp Glu Gln Arg Glu Cys Glu Asn Lys Ala Ala Ala Leu Ala
            580                 585                 590

Arg Ala Ile Asp Leu Ala Glu Ser Ala Phe Val Asp Lys Glu
        595                 600                 605


<210> 33
<211> 1821
<212> DNA
<213> Artificial Sequence

<220>
<223> Description of Artificial Sequence:Modified DNA
      sequence of Sequence No.8

<400> 33
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180
atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360
agcttcctct tcgagttcgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600
ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780

cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacatacgcc aatccatttg aagtctatcg agctttacga 1080
attgtgaacc caagtccata catggcatat gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380
tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560
ggagggatat catttgacgg agacatgctt atcgctcttg cactccgcac cattgtgttc 1620
tcaacagcgc caagccacaa cacgatgtac tcatacaaag acaccgagag gcgccgggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g 1821

<210> 34
<211> 1821
<212> DNA
<213> Artificial Sequence

<220>
<223> Description of Artificial Sequence:Modified DNA
      sequence of Sequence No.8

<400> 34
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180
atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360
agcttcctct tcgagtccgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600
ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780
cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacatacgcc aatccatttg aagtctatcg agctttacga 1080
attgtgaacc caagtccata catgctatat gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380
tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560
ggagggatat catttgacgg agacatgctt atcgctcttg cactccgcac cattgtgttc 1620
tcaacagcgc caagccacaa cacgatgtac tcatacaaag acaccgagag gcgccgggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g 1821

<210> 35
<211> 1821
<212> DNA
<213> Artificial Sequence

<220>
<223> Description of Artificial Sequence:Modified DNA
      sequence of Sequence No.8

<400> 35
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180
atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360
agcttcctct tcgagttcgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600
ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780
cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacatacgcc aatccatttg aagtctatcg agctttacga 1080
attgtgaacc caagtccata catggcatat gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380
tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560
ggagggatat catttgacgg agacatggac atcgcgctag cactccgcac cattgtgttc 1620
tcaacagcgc caagccacaa cacgatgtac tcatacaaag acaccgagag gcgcccggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g                                           1821

<210> 36
<211> 1821
<212> DNA
<213> Artificial Sequence

<220>
<223> Description of Artificial Sequence:Modified DNA
      sequence of Sequence No.8

<400> 36
atggagtcca tcgccgccgc cacgttcacg ccctcgcgcc tcgccgcccg ccccgccact 60
ccggcggcgg cggcggcccc ggttagagcg agggcggcgg tagcggcagg agggaggagg 120
aggacgagta ggcgcggcgg cgtgaggtgc tccgcgggga agccagaggc aagcgcggtg 180
atcaacggga gcgcggcggc gcgggcggcg gaggaggaca ggaggcgctt cttcgaggcg 240
gcggagcgtg ggagcgggaa gggcaacctg gtgcccatgt gggagtgcat cgtctccgac 300
cacctcaccc ccgtgctcgc ctaccgctgc ctcgtccccg aggacaacat ggagacgccc 360
agcttcctct tcgagtccgt cgagcagggg cccgagggca ccaccaacgt cggtcgctat 420
agcatggtgg gagcccaccc agtgatggag gtcgtggcaa aggagcacaa ggtcacaatc 480
atggaccacg agaagggcaa ggtgacggag caggtcgtgg atgatcctat gcagatcccc 540
aggagcatga tggaaggatg gcacccgcag cagatcgatc agctccccga ttccttcacc 600

ggtggatggg tcgggttctt ttcctatgat acagtccgtt atgttgaaaa gaagaagctg 660
cccttctccg gtgctcccca ggacgatagg aaccttcctg atgttcacct tgggctttat 720
gatgatgttc tcgtcttcga caatgtcgag aagaaagtat atgtcatcca ttgggtaaat 780
cttgatcggc atgcaaccac cgaggatgca ttccaagatg gcaagtcccg gctgaacctg 840
ttgctatcta aagtgcacaa ttcaaatgta cccaagcttt ctccaggatt tgtaaagtta 900
cacactcggc agtttggtac acctttgaac aaatcaacca tgacaagtga tgagtacaag 960
aatgctgtta tgcaggctaa ggagcatatt atggctggtg atattttcca gattgtttta 1020
agccagaggt ttgagaggcg aacatacgcc aatccatttg aagtctatcg agctttacga 1080
attgtgaacc caagtccata catgctatat gtacaggcaa gaggctgtgt cctggtagca 1140
tctagtccag aaattcttac tcgtgtgagg aagggtaaaa ttattaaccg tccacttgct 1200
gggactgttc gaaggggcaa gacagagaag gaagatgaaa tgcaagagca acagctacta 1260
agtgatgaaa aacagtgtgc tgaacatatt atgcttgtag atttgggaag gaatgatgtt 1320
ggaaaggtct ccaaacctgg atctgtgaag gtggagaaat taatgaacat tgaacgctac 1380
tcccatgtca tgcacatcag ttccacggtg agtggagagt tggatgatca tctccaaagt 1440
tgggatgccc tgcgagccgc gttgcctgtt ggaacagtta gtggagcacc aaaggtgaaa 1500
gccatggagc tgatagacga gctagaggtc acaagacgag gaccatacag tggcggcctt 1560
ggagggatat catttgacgg agacatggac atcgcgctag cactccgcac cattgtgttc 1620
tcaacagcgc caagccacaa cacgatgtac tcatacaaag acaccgagag gcgccgggag 1680
tgggtcgctc accttcaggc tggtgctggc attgtcgctg atagcagccc agacgacgag 1740
caacgtgaat gcgagaacaa ggcagccgct ctggctcgag ccatcgatct tgctgaatca 1800
gctttcgtag acaaggaata g 1821

<210> 37
<211> 31
<212> DNA
<213> Artificial Sequence

<220>
<223> Description of Artificial Sequence:Primer for
      site-directed mutagenesis

<400> 37
gcttcctctt cgagttcgtc gagcaggggc c 31

<210> 38
<211> 31
<212> DNA
<213> Artificial Sequence

<220>
<223> Description of Artificial Sequence:Primer for
      site-directed mutagenesis

<400> 38
ggcccctgct cgacgaactc gaagaggaag c 31

<210> 39
<211> 30
<212> DNA
<213> Artificial Sequence

<220>
<223> Description of Artificial Sequence:Primer for
      site-directed mutagenesis

<400> 39
caagtccata catgctatat gtacaggcaa 30

<210> 40

<211> 30
<212> DNA
<213> Artificial Sequence

<220>
<223> Description of Artificial Sequence:Primer for
      site-directed mutagenesis

<400> 40
ttgcctgtac atatagcatg tatggacttg                                        30


<210> 41
<211> 33
<212> DNA
<213> Artificial Sequence

<220>
<223> Description of Artificial Sequence:Primer for
      site-directed mutagenesis

<400> 41
aaaactagta tggagtccat cgccgccgcc acg                                    33


<210> 42
<211> 32
<212> DNA
<213> Artificial Sequence

<220>
<223> Description of Artificial Sequence:Primer for
      site-directed mutagenesis

<400> 42
aaagtcgact gagagagact ctattccttg tc                                     32


<210> 43
<211> 33
<212> DNA
<213> Artificial Sequence

<220>
<223> Description of Artificial Sequence:Artificially
      synthesized primer sequence

<400> 43
aaaactagta tggagtccat cgccgccgcc acg                                    33


<210> 44
<211> 32
<212> DNA
<213> Artificial Sequence

<220>
<223> Description of Artificial Sequence:Artificially
      synthesized primer sequence

<400> 44
aaagtcgact gagagagact ctattccttg tc                                     32

<210> 45
<211> 22
<212> DNA
<213> Artificial Sequence

<220>
<223> Description of Artificial Sequence:Artificially
      synthesized primer sequence

<400> 45
ggatggcacc cgcagcagat cg                                                22


<210> 46
<211> 23
<212> DNA
<213> Artificial Sequence

<220>
<223> Description of Artificial Sequence:Artificially
      synthesized primer sequence

<400> 46
gtactcatca cttgtcatgg ttg                                               23

## Description

### TECHNICAL FIELD

The present invention relates to a novel modified gene of rice anthranilate synthase gene OASA2 and use thereof, and particularly to a novel modified gene that encodes a modified rice anthranilate synthase having (i) enzyme activity that substantially matches or exceeds the enzyme activity of wild type rice anthranilate synthase and (ii) resistance to feedback inhibition by tryptophan, and use thereof.

### BACKGROUND ART

Tryptophan, one kind of amino acids constituting proteins, is essential to sustain functions of living organisms. Animals are unable to synthesize tryptophan and must resort to food as a source of tryptophan. Cereals such as rice, corn, and wheat have a significantly low tryptophan content, and as such cereal feedings generally need to be supplemented with industrially produced tryptophan. In the biosynthetic pathway of tryptophan, anthranilate is synthesized from chorismate. It is known that the synthesis of anthranilate involves the catalytic action of rice anthranilate synthase, and that the formation of anthranilate is followed by six-step enzyme reactions converting anthranilate to indole and to the final product tryptophan (see Non-Patent Publication 1).

The inventors of the present invention have found and isolated two alpha subunit genes OASA1 and OASA2 of rice anthranilate synthase (see Patent Publication 1). As a result of detailed study on characteristics of these genes, the inventors have reported that OASA2 was expressed more abundantly and encoded a protein that mainly functions as an alpha subunit of rice anthranilate synthase (see Non-Patent Publication 2). The inventors have also reported that OASA2 protein was highly susceptible to tryptophan concentration, and that activity of OASA2 protein was attenuated with increase in cellular concentration of tryptophan (see Non-Patent Publication 3).

Then, if functions of OASA2 protein could be modified, it would be possible to produce a new plant variety that can accumulate tryptophan in high concentration. The inventors have reported that, in the modification of rice anthranilate synthase gene, transformation of mutant OASA1 protein that had been rendered resistant to tryptophan feedback inhibition, for example, by the substitution an asparagine residue for the asparatate residue at position 323 of the first isozyme alpha subunit OASA1 protein (D323N) increased the level of free tryptophan by 180 fold in calluses, and 35 fold in recombinant rice, as compared with the wild type (see Non-Patent Publication 2).

Meanwhile, it is very time consuming and laborious to randomly introduce mutations in a gene and screen for functions of mutant protein encoded by the modified gene. Further, to simultaneously introduce more than one mutation at a target site using a random mutation introducing method is very difficult, if possible at all.
[Patent Publication 1]
   PCT International Publication WO99/11800
[Non-Patent Publication 1]
   Experimental Techniques in Biochemistry, Vol. 11, 1976,pp. 652-653, TOKYO KAGAKU DOZIN CO., LTD.
[Non-Patent Publication 2]
   Tozawa Y, Hasegawa H, Terakawa T and Wakasa K (2001) Characterization of rice anthranilate synthase alfa subunit genes OSASA1 and OSASA2: tryptophan accumulation in transgenic rice expressing a feedback-insensitive mutant of OASA1. Plant Physiology 126: 1493-1506
[Non-Patent Publication 3]
   Takuya Kanno, Koji Kasai, Yasuko Ikejiri-Kanno, Kyo Wakasa and Yuzuru Tozawa (2004) In vitro reconstitution of rice anthranilate synthase: distinct functional properties of the alpha subunits OASA1 and OASA2. Plant Molecular Biology 54: 11-22

### DISCLOSURE OF INVENTION

As described above, cereal feedings are supplemented with industrially produced tryptophan. Due to the relatively high price of tryptophan compared with other amino acids, there has been demand for production of cereals with a high tryptophan content. Production of a new plant variety that can accumulate tryptophan in high concentration is possible by introducing mutation into rice anthranilate synthase gene OASA2 and thereby modify functions of OASA2 protein in such a manner that there will be no attenuation of enzyme activity even under high intracellular concentrations of tryptophan.

The present invention was made in view of the foregoing problems, and an object of the present invention is to realize a new plant variety with a high-concentration tryptophan content, which is realized by providing a rice anthranilate synthase whose functions have been modified to maintain enzyme activity even under high intracellular concentrations of tryptophan, and a novel modified gene that encodes such an enzyme.

The inventors of the present invention diligently worked to solve the foregoing problems, and produced a protein having resistance to tryptophan feedback inhibition by introducing mutation to rice anthranilate synthase gene OASA2. Further, in accomplishing the present invention, the inventors introduced more than one mutation to wild type rice anthranilate synthase gene OASA2, and from a combination of these mutations, produced a protein that had enzyme activity substantially matching or exceeding that of wild type rice anthranilate synthase, in addition to resistance to tryptophan feedback inhibition.

Specifically, a polypeptide according to the present invention has a mutation at at least one of position 126, 367, and 369 of an amino acid sequence of SEQ ID NO: 1, wherein the polypeptide has resistance to tryptophan feedback inhibition in a biosynthetic pathway of tryptophan.

Preferably, the polypeptide also has a mutation at at least one of position 351, 522, and 530 of the amino acid sequence of SEQ ID NO: 1, and enzyme activity at least 0.7 times enzyme activity of wild type rice anthranilate synthase. The polypeptide having resistance to tryptophan feedback inhibition and enzyme activity at least 0.7 times enzyme activity of wild type rice anthranilate synthase is able to synthesize tryptophan even under high intracellular concentrations of tryptophan, and plants expressing such a polypeptide are useful as they contain tryptophan in high concentration and therefore have high nutritional values.

It is preferable that the mutation at position 126 of the amino acid sequence of SEQ ID NO: 1 be a substitution of phenylalanine for serine, that the mutation at position 367 of the amino acid sequence of SEQ ID NO: 1 be a substitution of alanine or isoleucine for tyrosine, and that the mutation at position 369 of the amino acid sequence of SEQ ID NO: 1 be a substitution of leucine for alanine. Further, it is preferable that the mutation at position 351 of the amino acid sequence of SEQ ID NO: 1 be a substitution of asparatate for asparagine, that the mutation at position 522 of the amino acid sequence of SEQ ID NO: 1 be a substitution of tyrosine for glycin, and that the mutation at position 530 of the amino acid sequence of SEQ ID NO: 1 be a substitution of alanine or asparatate for leucine. With these amino acid substitutions, a mutant rice anthranilate synthase is realized that has resistance to tryptophan feedback inhibition, or a mutant rice anthranilate synthase is realized that has resistance to tryptophan feedback inhibition and enzyme activity at least 0.7 times the enzyme activity of wild type rice anthranilate synthase.

The mutant rice anthranilate synthase may be a polypeptide with an amino acid sequence of any one of SEQ ID NOs: 2 through 7 and SEQ ID NOs: 29 through 32, or a polypeptide with an amino acid sequence with a deletion, substitution, or addition of one or more amino acids in an amino acid sequence of any one of SEQ ID NOs: 2 through 7 and SEQ ID NOs: 29 through 32. With these amino acid sequences, a mutant rice anthranilate synthase is realized that has resistance to tryptophan feedback inhibition, or a mutant rice anthranilate synthase is realized that has resistance to tryptophan feedback inhibition and enzyme activity at least 0.7 times the enzyme activity of wild type rice anthranilate synthase.

Further, a polypeptide according to the present invention has a mutation in a tryptophan binding region of rice anthranilate synthase, the mutation occurring at position 5 of an amino acid sequence of SEQ ID NO: 26, and the polypeptide having resistance to tryptophan feedback inhibition in a biosynthetic pathway of tryptophan. Preferably, the mutation at position 5 of the amino acid sequence of SEQ ID NO: 26 is a substitution of alanine or isoleucine for tyrosine. The polypeptide having resistance to tryptophan feedback inhibition is able to synthesize tryptophan even under high intracellular concentrations of tryptophan, and plants expressing such a polypeptide are useful as they contain tryptophan in high concentration and therefore have high nutritional values.

A polynucleotide according to the present invention encodes a polypeptide according to the present invention. Preferably a polynucleotide according to the present invention has a base sequence of any one of SEQ ID NOs: 9 through 14 and SEQ ID NOs: 33 through 36, or a base sequence that hybridizes under stringent conditions with a base sequence complementary to the base sequence of any one of SEQ ID NOs: 9 through 14 and SEQ ID NOs: 33 through 36. By introducing the polynucleotide in cells, a transformant can be produced that can express a polypeptide according to the present invention in the cell.

A marker gene for screening transformants according to the present invention comprises a polynucleotide according to the present invention. A polypeptide encoded by a polynucleotide according to the present invention confers resistance to a tryptophan-like compound in cells expressing the polypeptide. The polynucleotide can therefore be used as a marker gene for screening for a transformant expressing resistance to a tryptophan-like compound.

A recombinant expression vector according to the present invention comprises a polynucleotide according to the present invention. A recombinant expression vector according to the present invention can be used as a recombinant expression vector for introducing a polynucleotide according to the present invention to cells. When a polynucleotide according to the present invention is used as a selection marker, a recombinant expression vector can also be used as a recombinant expression vector for introducing other genes into cells.

A transformant according to the present invention incorporates therein a polynucleotide according to the present invention or a recombinant expression vector according to the present invention, and expresses a polypeptide that has resistance to tryptophan feedback inhibition in a biosynthetic pathway of tryptophan. Preferably, a transformant according to the present invention is a plant cell or a plant. Transgenic plants expressing the polypeptide that has resistance to tryptophan feedback inhibition are useful as they contain tryptophan in high concentration and therefore have high nutritional values. The present invention includes seeds obtained from such transgenic plants.

A method for screening transformed cells according to the present invention includes the steps of: introducing into cells a marker gene according to the present invention or a recombinant expression vector according to the present invention so as to render the cells resistant to a tryptophan-like compound that inhibits proliferation of cells; and screening for cells expressing resistance to the tryptophan-like compound. Use of the gene as a selection marker solves the problem of limited types of markers available for rice and other monocots. Further, use of the gene also solves the problem of limited types of selection markers available for introducing more than one gene into a cell. Specifically, cells that have incorporated more than one target gene can be screened for based on resistance to a tryptophan-like compound such as 5-methyltryptophan, in addition to resistance to an antibiotic such as hygromycin, which is commonly used as a selection marker. Further, since the marker gene originates in rice, it is expected that a protein encoded by the gene in the transformed rice have low antigenic activity.

A transformation kit according to the present invention includes a polynucleotide according to the present invention, or a recombinant expression vector according to the present invention. A transformation kit according to the present invention can be used to conveniently and efficiently obtain a transformant expressing a polypeptide according to the present invention.

A screening method according to the present invention is a method for screening for a substance that binds to at least one of a polypeptide according to the present invention and a wild type rice anthranilate synthase, and the method includes the steps of: screening for a substance binding to a polypeptide according to the present invention; screening for a substance binding to a wild type rice anthranilate synthase; and comparing results of the screening steps. A screening method according to the present invention allows for screening of a substance involved in tryptophan feedback inhibition in a biosynthetic pathway of tryptophan. A screening method according to the present invention can therefore produce mutant rice anthranilate synthase with enhanced resistance to the feedback inhibition.

A screening kit according to the present invention is a kit for performing a screening method according to the present invention, and includes a wild type rice anthranilate synthase and at least one of polypeptides according to the present invention. A screening kit according to the present invention can be used to perform a screening method according to the present invention both conveniently and efficiently.

As described above, a polypeptide according to the present invention has resistance to tryptophan feedback inhibition in a synthetic pathway of tryptophan, and enzyme activity substantially matching or exceeding that of wild type rice anthranilate synthase. A polypeptide according to the present invention can therefore synthesize tryptophan even under high concentrations of tryptophan.

A polynucleotide according to the present invention encodes a polypeptide according to the present invention. Thus, by introducing a polynucleotide according to the present invention into a plant cell, a transgenic plant can be produced that can synthesize tryptophan even under high concentrations of tryptophan.

A transgenic plant according to the present invention is useful as it contains tryptophan in high concentration and therefore provides food and feedings having superior nutritional values. Further, by producing feeding rice having a high tryptophan content, the cost of livestock feedings can be reduced. Further, the self-sufficiency rate of feedings can be increased through efficient use of paddy fields.

Additional objects, features, and strengths of the present invention will be made clear by the description below. Further, the advantages of the present invention will be evident from the following explanation in reference to the drawings.

### BRIEF DESCRIPTION OF DRAWINGS

- Figure 1: is a graph representing a result of measurement on anthranilate synthase activity in a measurement system using 100 mM NH₄Cl as an amide group donating substrate, showing mutant OASA2 proteins that had enhanced enzyme activity.
- Figure 2: is a graph representing a result of measurement on anthranilate synthase activity in a measurement system using 100 mM NH₄Cl as an amide group donating substrate, showing mutant OASA2 proteins that had resistance to tryptophan feedback inhibition.
- Figure 3: is a graph representing degrees of resistance of wild type and mutant proteins to tryptophan feedback inhibition.
- Figure 4(A): is a schematic diagram of wild type OASA2 protein.
- Figure 4(B): is a diagram comparing amino acid sequences in tryptophan binding regions of organisms having a tryptophan synthesis system.
- Figure 5: is a diagram schematizing recombinant vectors for introducing exogenous genes, showing each vector with wild type (wt) OASA2 gene or mutant OASA2 gene (Y367A, Y367A/L530D, S126F/L530D) used for transformation of a rice callus using an Agrobacterium method.

### BEST MODE FOR CARRYING OUT THE INVENTION

The following will describe one embodiment of the present invention. It should be appreciated however that the invention is not limited in any way by the following description.

### (1) Polypeptide according to the Present Invention

A polypeptide according to the present invention has a mutation in a tryptophan binding region of rice anthranilate synthase, the mutation occurring at position 5 of the amino acid sequence of SEQ ID NO: 26, and the polypeptide having resistance to feedback inhibition by tryptophan in a biosynthetic pathway of tryptophan. The mutation at position 5 of the amino acid sequence of SEQ ID NO: 26 is preferably a substitution of alanine or isoleucine for tyrosine.

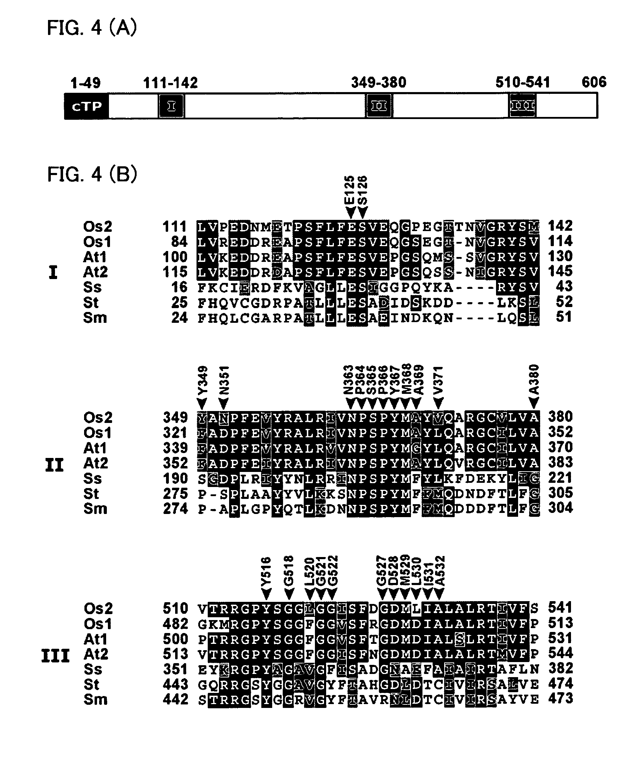

Figure 4(A) schematizes a rice anthranilate synthase (hereinafter, also referred to as "OASA2 protein" or "wild type OASA2 protein") encoded by a rice anthranilate synthase gene OASA2 (hereinafter, also referred to as "OASA2 gene"). In Figure 4(A), numbers represent positions of amino acids. Indicated by cTP is a chloroplast transit signal, and I, II, and III are domains of amino acid residues in tryptophan binding regions. Figure 4(B) compares amino acid sequences in the tryptophan binding regions I, II, and III of organisms with various types of tryptophan synthesis systems. In Figure 4(B), Os1 is rice OASA1 (Accession no. AB022602), Os2 is rice OASA2 (Accession no. AB022603), At1 is *Arabidopsis* ASA1 (Accession no. M92353), At2 is *Arabidopsis* ASA2 (Accession no. M92354), Ss is thermophilic archaebacteria (*Sulfolobus solfataricus*) TrpE (Accession no. 1QDL_A), St is *Salmonella* (*Salmonella tryphimurium*) TrpE (Accession no. 1I1Q_A), Sm is *Serratia* (*Serratia marcescens*) TrpE (Accession no. 1I7Q_A).

As can be seen in Figure 4(A) and Figure 4(B), the amino acid sequence (NPSPYM) of SEQ ID NO: 26 is conserved in the tryptophan binding region II in all of the organisms. This particular amino acid sequence is therefore believed to play a very important role in the tryptophan binding region. Other than the biological species as exemplified in Figure 4(B), the following non-limiting examples of biological species are known to have such conserved amino acid sequences: *Catharanthus roseus* α subunit (Accession no. CAC29060), *Ruta graveolens* ASα1 (Accession no. L34343), *Ruta graveolens* ASα2 (Accession no. L34344), tobacco ASA2 (Accession no. T01990), yeast (*Saccharomyces cerevisiae*) TRP2 (Accession no. X68327), *Escherichia coli* TrpE (Accession no. V00368), *Bacillus subtilis* TrpE (Accession no. P03963).

The amino acid sequence of SEQ ID NO: 26 corresponds to, for example, position 363 to 367 of SEQ ID NO: 1, which represents the amino acid sequence of wild type OASA2 protein encoded by rice anthranilate synthase gene OASA2. A substitution of tyrosine with other amino acids, preferably alanine or isoleucine in the conserved amino acid sequence of SEQ ID NO: 26 confers resistance to tryptophan feedback inhibition.

A polypeptide according to the present invention has a mutation in at least one of position 126, 367, and 369 of the amino acid sequence of SEQ ID NO: 1, and has resistance to tryptophan feedback inhibition in the biosynthetic pathway of tryptophan. A polypeptide with the amino acid sequence of SEQ ID NO: 1 is a wild type rice anthranilate synthase encoded by rice anthranilate synthase gene OASA2. More specifically, a polypeptide according to the present invention has a mutation in at least one of position 126, 367, and 369 of wild type rice anthranilate synthase (wild type OASA2 protein) encoded by rice anthranilate synthase gene OASA2 (OASA2 gene), and has resistance to tryptophan feedback inhibition. The type of mutation is not particularly limited, and it may be a substitution, a deletion, or an addition of amino acid, for example. For convenience of explanation, OASA2 protein will be referred to as "mutant OASA2 protein" if it has a mutation, regardless of the position, type, and number of mutations, among other things.

In the biosynthetic pathway of tryptophan in plants, tryptophan production proceeds by the production of anthranilate from chorismate, followed by six-step enzyme reactions converting anthranilate to indole and to the final product tryptophan (see Non-Patent Publication 1). In the pathway, the production of anthranilate from chorismate is catalyzed by the rice anthranilate synthase. Under the feedback inhibition by the final product tryptophan, the enzyme activity of the rice anthranilate synthase attenuates with a rise in concentration of tryptophan in the cell. As a consequence, there will be no synthesis of tryptophan when the accumulation of tryptophan in the cell reaches a certain level. As described above, a polypeptide according to the present invention has resistance to tryptophan feedback inhibition. This enables synthesis of tryptophan even under increased tryptophan concentrations in the cell, thereby producing plants with a high tryptophan content.

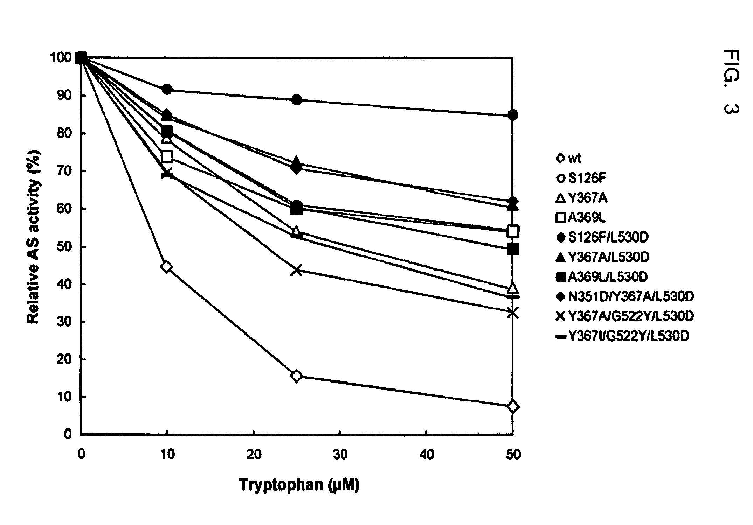

Resistance to tryptophan feedback inhibition can be measured using, for example, an *in vitro* enzyme activity measurement system, in which enzyme activity of wild type OASA2 protein is measured and resistance to tryptophan feedback inhibition is determined based on a ratio (in percent) of enzyme activity with tryptophan, with respect to 100% enzyme activity without tryptophan. More specifically, as shown in Figure 3, a comparison is made between wild type OASA2 protein and mutant OASA2 proteins with regard to a ratio (in percent) of enzyme activity with tryptophan with respect to 100% enzyme activity without tryptophan. The mutant OASA2 protein can be said to have resistance to tryptophan feedback inhibition when the measured enzyme activity with tryptophan (%) exceeds that of the wild type OASA2 protein. The degree by which the measured enzyme activity with tryptophan (%) of the mutant OASA2 protein exceeds that of the wild type OASA2 protein is not particularly limited, but the difference is preferably at least 2 fold, more preferably at least 3 fold, even more preferably at least 4 fold, and most preferably at least 5 fold.

More specifically, for example, in the measurement of enzyme activity using the measurement system described under (2) <Enzyme Activity Measurement 1> in Example 3 to be described later, the enzyme activity (anthranilate yield) with 100 µM tryptophan is preferably at least 20%, more preferably at least 30%, even more preferably 40%, and most preferably 50%, with respect to 100% enzyme activity (anthranilate yield) without tryptophan.

The amino acid that replaces the serine at position 126 of the OASA2 protein is not particularly limited as long as it satisfies the requirement of resistance to the feedback inhibition. Phenylalanine is preferable. In other words, a polypeptide with the amino acid sequence of SEQ ID NO: 29 (substitution of phenylalanine for serine at position 126), or a polypeptide with by the deletion, substitution, or addition of one or more amino acids in the amino acid sequence of SEQ ID NO: 29, having resistance to tryptophan feedback inhibition, is preferable.

The amino acid that replaces the tyrosine at position 367 of the OASA2 protein is not particularly limited as long as it satisfies the requirement of resistance to the feedback inhibition. Alanine, isoleucine, phenylalanine, or valine is preferable. Alanine or isoleucine is more preferable. Specifically, a polypeptide with the amino acid sequence of SEQ ID NO: 2 (substitution of alanine for tyrosine at position 367), a polypeptide with the amino acid sequence of SEQ ID NO: 3 (substitution of isoleucine for tyrosine at position 367), or a polypeptide with the deletion, substitution, or addition of one or more amino acids in the amino acid sequence of SEQ ID NO: 2 or 3, having resistance to tryptophan feedback inhibition, is preferable.

The amino acid that replaces the alanine at position 369 of the OASA2 protein is not particularly limited as long as it satisfies the requirement of resistance to the feedback inhibition. Leucine is preferable. Specifically, a polypeptide with the amino acid sequence of SEQ ID NO: 30 (substitution of leucine for alanine at position 369), or a polypeptide with the deletion, substitution, or addition of one or more amino acids in the amino acid sequence of SEQ ID NO: 30, having resistance to tryptophan feedback inhibition, is preferable.

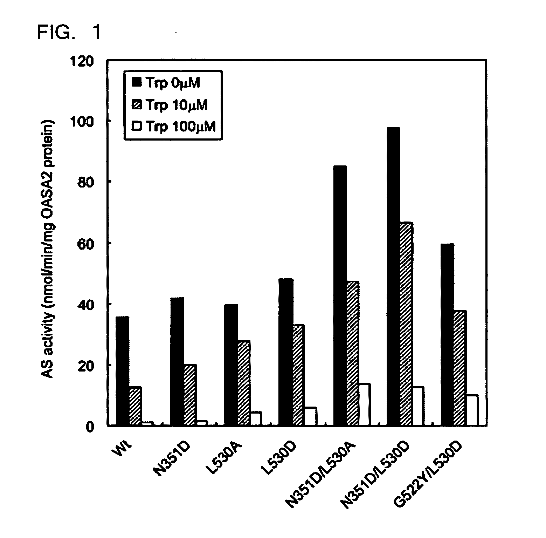

A polypeptide according to the present invention preferably has a mutation at at least one of position 351, 522, and 530 of the OASA2 protein, in addition to a mutation at at least one of position 126, 367, or 369 of the OASA2 protein. The inventors have confirmed that the mutant OASA2 protein with one or more mutations at position 351, 522, and 530 has improved enzyme activity as compared with the wild type OASA2 protein (see Figure 1).

By combining the mutation that confers enhanced enzyme activity with mutation that confers resistance to tryptophan feedback inhibition, a mutant OASA2 protein can be obtained that has both resistance to tryptophan feedback inhibition and enzyme activity substantially matching or exceeding that of the wild type OASA2 protein. As used herein, "enzyme activity" refers to, for example, an anthranilate yield as measured by the activity measurement system described in (2) <Enzyme Activity Measurement 1> in Example 3 to be described later. However, a method of measuring enzyme activity is not just limited to this example. Any conventional measurement method of enzyme activity, or modifications thereof, can be used that are adapted to measure activity of rice anthranilate synthase.

Further, as used herein, "enzyme activity substantially matching or exceeding that of the wild type OASA2 protein" means that, in an *in vitro* enzyme activity measurement system (no tryptophan) for example, the enzyme activity of the mutant OASA2 protein is preferably at least 0.7 times, more preferably at least 0.8 times, even more preferably at least 0.9 times, or most preferably equal to or greater than the enzyme activity of the wild type OASA2 protein. More specifically, for example, in the measurement of enzyme activity using the activity measurement system (no tryptophan) described in (2) <Enzyme Activity Measurement 1> in Example 3 to be described later, the anthranilate yield by the mutant OASA2 protein is preferably at least 0.7 times, more preferably at least 0.8 times, even more preferably at least 0.9 times, or most preferably equal to or greater than the anthranilate yield by the wild type OASA2 protein.

Position 351, 522, and 530 of the OASA2 protein are asparagine, glycin, and leucine, respectively. The amino acids that replace these amino acids are not particularly limited as long as the mutations, occurring either alone or in combination, coupled to at least one mutation occurring at position 126, 367, and 369 confer (1) resistance to tryptophan feedback inhibition and (2) enzyme activity at least 0.7 times the enzyme activity of the wild type OASA2 protein. The amino acid that replaces the asparagine at position 351 is preferably asparatate, and the amino acid that replaces the glycin at position 522 is preferably tyrosine. The amino acid that replaces the leucine at position 530 is preferably alanine or asparatate, or more preferably asparatate.

More specifically, a polypeptide with a combination of the following mutations is preferable.
(i) A polypeptide with an alanine-for-tyrosine substitution at position 367, and an asparatate-for-leucine substitution at position 530 (SEQ ID NO: 4).
(ii) A polypeptide with an asparatate-for-asparagine substitution at position 351, an alanine-for-tyrosine substitution at position 367, and an asparatate-for-leucine substitution at position 530 (SEQ ID NO: 5).
(iii) A polypeptide with an alanine-for-tyrosine substitution at position 367, a tyrosine-for-glycine substitution at position 522, and an asparatate-for-leucine substitution at position 530 (SEQ ID NO: 6).
(iv) A polypeptide with an isoleucine-for-tyrosine substitution at position 367, a tyrosine-for-glycine substitution at position 522, and an asparatate-for-leucine substitution at position 530 (SEQ ID NO: 7).
(v) A polypeptide with a leucine-for-alanine substitution at position 369 (SEQ ID NO: 30).
(vi) A polypeptide with a phenylalanine-for-serine substitution at position 126, and an asparatate-for-leucine substitution at position 530 (SEQ ID NO: 31).
(vii) A polypeptide with a leucine-for-alanine substitution at position 369, and an asparatate-for-leucine substitution at position 530 (SEQ ID NO: 32).

Among these polypeptides, the polypeptide (vi) is most preferable. This is because introduction of a coding gene of this polypeptide into a rice callus increased the free tryptophan content by about 400 fold, as will be described later in Examples.

More specifically, a polypeptide according to the present invention is preferably a polypeptide with the amino acid sequence of any one of SEQ ID NOs: 4 through 7 and SEQ ID NOs: 30 through 32, or a polypeptide with the deletion, substitution, or addition of one or more amino acids in the amino acid sequence of any one of SEQ ID NOs: 4 through 7 and SEQ ID NOs: 30 through 32, having resistance to tryptophan feedback inhibition, and enzyme activity at least 0.7 times the enzyme activity of the wild type OASA2 protein.

As used herein, the "deletion, substitution, or addition of one or more amino acids" means deletion, substitution, or addition of amino acids in numbers (for example, no greater than 20, preferably no greater than 10, more preferably no greater than 7, even more preferably no greater than 5, and particularly preferably no greater than 3) that can be brought about by known mutant polypeptide producing methods such as site-directed mutagenesis. Such mutant polypeptides are not just limited to those in which mutations have been artificially induced using known mutation polypeptide producing methods, but include those isolated and purified from similar mutant polypeptides that exist in nature.

A polypeptide according to the present invention is formed of amino acids joined together by peptide bonding. However, a polypeptide according to the present invention is not just limited to this example and may include non-polypeptide structures. Non-limiting examples of such non-polypeptide structures include sugar chains and isoprenoid groups.

A polypeptide according to the present invention may include additional polypeptides. For example, the polypeptide may be epitope-labeled with His, Myc, or Flag.

Further, a polypeptide according to the present invention may be expressed intracellularly by being encoded by a polynucleotide according to the present invention (polynucleotide encoding a polypeptide according to the present invention) that has been introduced into a host cell. Alternatively, a polypeptide according to the present invention may be isolated and purified from cells or tissues. Further, depending upon expression conditions in the host cell, a polypeptide according to the present invention may be a fusion protein fused together with other polypeptides. Further, a polypeptide according to the present invention may be chemically synthesized.

A method by which a polypeptide according to the present invention is obtained (producing method) is not particularly limited. However, since a polypeptide according to the present invention is a variant of wild type OASA2 protein having incorporated therein one or more mutations, a polypeptide according to the present invention is optimally produced first by preparing a mutant gene in which mutation has been artificially introduced into the base sequence of OASA2 gene, and then expressing the polypeptide encoded by the mutant gene. The base sequence can be mutated by a known method, for example, by the Kunkel method or with the use of PCR, as used by the inventors in Examples below. Alternatively, a commercially available kit may be used (for example, Mutan-K, Mutan-Super Express Km, and LA-PCR *in vitro* mutagenesis Kit, all products of TAKARA BIO INC.). That is, a polypeptide according to the present invention can be obtained by introducing OASA2 gene, that has been artificially mutated, into a suitable expression vector, and then introducing the expression vector into a suitable host cell and obtaining the product of translation (polypeptide) in the host cell. Alternatively, a product (polypeptide) of mutated OASA2 gene may be obtained using a known acellular protein synthesis system such as a wheat embryo acellular system, as used by the inventors in Examples below. One example of a method for obtaining a polypeptide according to the present invention will be described in Examples.

### (2) Polynucleotide According to the Present Invention

A polynucleotide according to the present invention encodes a polypeptide according to the present invention. For example, a polynucleotide according to the present invention encodes polypeptides as defined in [1] and [2] below. Note that, no further explanation is given for a polypeptide according to the present invention since it was described in detail in Section (1) above.
[1] A polypeptide with a mutation at at least one of position 126, 367, and 369 of the amino acid sequence of SEQ ID NO: 1, having resistance to tryptophan feedback inhibition in a biosynthetic pathway of tryptophan.
[2] A polypeptide with a mutation at at least one of position 351, 522, and 530 of the amino acid sequence of SEQ ID NO: 1 in addition to the mutation in [1], having (1) resistance to tryptophan feedback inhibition in a biosynthetic pathway of tryptophan, and (2) enzyme activity at least 0.7 times the enzyme activity of wild type rice anthranilate synthase.
   For example, a polynucleotide that encodes the amino acid sequence of SEQ ID NO: 2, i.e., a polypeptide with an alanine-for-tyrosine substitution at position 367 of the amino acid sequence of OASA2 protein may be a polynucleotide in which the bases (tac: tyrosine) at position 1099, 1100, and 1101 in the base sequence of SEQ ID NO: 8 (base sequence in an open reading frame region of OASA2 gene) have been mutated to a codon, namely gct, gcc, or gcg, that corresponds to alanine. The base sequence other than position 1099 to 1101 may be different from the base sequence of SEQ ID NO: 8. For example, the polynucleotide may have base substitutions that do not cause mutation in the encoded amino acid.
   The same applies to a polynucleotide that encodes a polypeptide having amino acid mutations other than those exemplified above. Specifically, a polynucleotide according to the present invention may be a polynucleotide in which three bases in the base sequence of SEQ ID NO: 8, corresponding in position to a mutated amino acid are replaced with bases of a codon that corresponds to the substituted amino acid, or a polynucleotide with non-mutating base substitutions in part of the base sequence where the foregoing base mutations do not occur.
   A polynucleotide according to the present invention preferably encodes polypeptides as defined in [3] and [4] below.
[3] A polypeptide with the amino acid sequence of SEQ ID NO: 2, 3, 29, or 30, or a polypeptide with the deletion, substitution, or addition of one or more amino acids in the base sequence of SEQ ID NO: 2, 3, 29, or 30, having resistance to tryptophan feedback inhibition.
[4] A polypeptide with the amino acid sequence of any one of SEQ ID NOs: 4 through 7 and SEQ ID NOs: 30 through 32, or a polypeptide with the deletion, substitution, or addition of one or more amino acids in the amino acid sequence of SEQ ID NOs: 4 through 7 and SEQ ID NOs: 30 through 32, having (1) resistance to tryptophan feedback inhibition, and (2) enzyme activity at least 0.7 times the enzyme activity of the wild type OASA2 protein.

That is, the polynucleotide is not limited to a specific sequence as long as the base sequence encodes any of the amino acid sequences of SEQ ID NOs: 2 through 7 and SEQ ID NOs: 29 through 32. It is however preferable that the polynucleotide be highly homologous to a polynucleotide with the base sequence of SEQ ID NO: 8, i.e., the base sequence in an open reading frame region of OASA2 gene. For example, the polynucleotide has at least 90% identity, preferably at least 95% identity, and most preferably at least 97% identity.

As a non-limiting example, a polynucleotide according to the present invention may be a polynucleotide that hybridizes under stringent conditions with a polynucleotide having a complementary base sequence with the base sequence of a polynucleotide as represented by any one of SEQ ID NOs: 9 through 14 and SEQ ID NOs: 33 through 36, or the base sequence of a polynucleotide as represented by any one of SEQ ID NOs: 9 through 14 and SEQ ID NOs: 33 through 36. A polynucleotide with the base sequence of any of SEQ ID NOs: 9 through 14 and SEQ ID NOs: 33 through 36 respectively corresponds to a polynucleotide with the base sequence that encodes a polypeptide with the amino acid sequence of any of SEQ ID NOs: 2 through 7 and SEQ ID NOs: 29 through 32. A polynucleotide with the base sequence of any of SEQ ID NOs: 9 through 14 and SEQ ID NOs: 33 through 36 is a polynucleotide that was produced by the inventors using methods described in the Examples.

As used herein, "under stringent conditions" means that hybridization occurs only when there is at least 90% identity, preferably at least 95% identity, and most preferably at least 97% identity between sequences. For example, it means binding under washing conditions 2 × SSC at 60°C.

Hybridization can be preformed using known methods, for example, such as one described in "Molecular Cloning (Third Edition)" (J. Sambrook & D. W. Russell, Cold Spring Harbor Laboratory Press, 2001). As a rule, stringency increases with increase in temperature and with decrease in salt concentration.

A polynucleotide according to the present invention includes DNA and RNA, which may be single stranded or double stranded. Further, a polynucleotide according to the present invention may include sequences of untranslated regions (UTR) or vector sequences (including a sequence of expression vector).

A method by which a polynucleotide according to the present invention is obtained (producing method) is not particularly limited. For example, a method that artificially introduces mutation in the base sequence of OASA2 gene can be used. The base sequence can be mutated by a known method, for example, by the Kunkel method or with the use of PCR. Alternatively, a commercially available kit may be used (for example, Mutan-K, Mutan-Super Express Km, and LA-PCR *in vitro* mutagenesis Kit, all products of TAKARA BIO INC.). One example of a method for obtaining a polynucleotide according to the present invention will be described in detail later in Examples.

### (3) A Marker Gene for Transformation and Screening Method of Transformed Cells

A polynucleotide according to the present invention can be used as a marker gene for transformation. Specifically, a marker gene for screening transformants according to the present invention comprises a polynucleotide according to the present invention as described in Section (2) above. Cells having incorporated therein a polynucleotide according to the present invention show resistance to tryptophan. Thus, using a medium that has been supplemented with an analog compound of tryptophan, it is possible to screen for only cells that have incorporated the polynucleotide. Following is some examples of tryptophan-like compounds that can be used as screening agents to inhibit cell growth: 5-methyltryptophan (5MT), 4-methyltryptophan (4MT), 6-methyltryptophan (6MT), 7-methyltryptophan (7MT), 6-methylanthranilate (6MA), 5-methylanthranilate (5MA), 3-methylanthranilate (3MA), 5-fluoroanthranilate (5FA), and 6-fluoroanthranilate (6FA).

Specifically, a polynucleotide according to the present invention can be used as a marker gene for transformation, for example, by constructing an expression vector that has incorporated therein the polynucleotide, and then introducing the expression vector into a target cell. Cells that have incorporated the expression vector and expressing the polypeptide encoded by the polynucleotide according to the present invention acquire resistance to tryptophan, and can grow in medium supplemented with the screening agents as exemplified above, whereas cells that did not incorporate the expression vector or not expressing the polypeptide encoded by the polynucleotide according to the present invention show inhibited cell growth. That is, it is possible to screen for only transformed cells that have incorporated therein the expression vector and expressing the polypeptide encoded by the polynucleotide according to the present invention. In this example, a polynucleotide according to the present invention serves not only as a marker gene but as a gene that is expressed in the transformed cells. However, a polynucleotide according to the present invention can be used only as a marker gene. Further, using a transcription promoter specific to plant callus cells for example, it is possible to control expression time of a polynucleotide according to the present invention used as a selection marker. In this case, another expression vector is constructed that has incorporated therein a coding gene of a protein to be expressed in the target cell. The expression vector can then be used to transform the target cell. Further, instead of constructing an expression vector that has incorporated therein a polynucleotide according to the present invention, a polynucleotide according to the present invention can be solely introduced into a target cell.

Use of a polynucleotide according to the present invention as a selection marker can solve the problem of limited types of markers available in rice and other monocots. Further, use of the gene can also solve the problem of limited types of selection markers that can be used to introduce a plurality of genes into a cell. Specifically, cells that have incorporated more than one target gene can be screened for based on resistance to a tryptophan-like compound such as 5-methyltryptophan, in addition to resistance to an antibiotic such as hygromycin, which is commonly used as a selection marker. Further, since the marker gene originates in rice, it is expected that a protein encoded by the gene in the transformed rice have low antigenic activity.

Note that, the present invention also includes a screening method of transformed cells, the method introducing a marker gene according to the present invention or a recombinant expression vector (described later) into cells to render cells resistant to tryptophan-like compounds that inhibit cell growth, and then screening for cells expressing resistance to the tryptophan-like compounds.

### (4) Recombinant Expression Vector and Transformation Kit

A recombinant expression vector according to the present invention is not particularly limited as long as it includes a polynucleotide according to the present invention described in Section (2) above. A recombinant expression vector with inserted cDNA is one example. A recombinant expression vector according to the present invention can be produced using a plasmid, a phage, or a cosmid. These are merely examples and any conventional methods can be used.

The type of vector is not particularly limited and a vector that can be expressed in a host cell is suitably selected. Specifically, a promoter sequence for reliable expression of the gene is suitably selected according to the type of host cell, and the promoter sequence is incorporated in various kinds of plasmids with a polynucleotide according to the present invention to provide an expression vector.

A recombinant expression vector according to the present invention can be used to express a polypeptide according to the present invention. A recombinant expression vector according to the present invention can also be used to express proteins encoded by other genes that have been incorporated in the recombinant expression vector, using a polynucleotide according to the present invention as a marker gene.

Various markers can be used to confirm whether a polynucleotide according to the present invention has been incorporated in host cells or successfully expressed in host cells. For example, using a drug-resistant gene as a marker that renders cells resistant to antibiotics such as hygromycin, a plasmid or the like including the marker and a polynucleotide according to the present invention is introduced into host cells as an expression vector. Whether the gene of the present invention has been incorporated or not can be confirmed from the expression of the marker gene.

The host cells are not particularly limited as long as they are cells or organisms with a tryptophan synthesis system. Various types of conventional cells can be suitably used. Specifically, some of the non-limiting examples include bacteria such as *Escherichia coli,* and yeasts such as *Saccharomyces cerevisiae* and *Schizosaccharomyces pombe.*

A method of introducing the expression vector into host cells, i.e., a transformation method, is not particularly limited either. Conventional methods such as an electroporation method, a calcium phosphate method, a protoplast method, and a lithium acetate method can be suitably used.

A transformation kit according to the present invention includes at least one of polynucleotides according to the present invention described in Section (2) above, and a recombinant expression vector according to the present invention. The rest of the arrangement is not particularly limited, and the kit may additionally include reagents, instruments, and the like as suitably selected as needed. A transformation kit can be used to conveniently and efficiently obtain transformed cells.

### (5) Transformant

A transformant according to the present invention is not particularly limited as long as it has incorporated therein a polynucleotide according to the present invention described in Section (2) above or a recombinant expression vector described in Section (4), and in which a polypeptide having resistance to tryptophan feedback inhibition in the biosynthetic pathway of tryptophan is expressed. As used herein the term "transformant" means not only cells, tissues, and organs but also individual organisms themselves.

A method for forming (producing) the transformant is not particularly limited. For example, the recombinant expression vector may be introduced into a host cell to produce a transformant. The type of transformed organism is not particularly limited as long as it is a cell or organism with a tryptophan synthesis system. For example, various kinds of microorganisms as exemplified above as host cells in Section (4) can be used.

A transformant according to the present invention is preferably a plant cell or a plant. A transformed plant according to the present invention has a high tryptophan content, and therefore has additional values as a food material or feeding with high nutritional values.

The recombinant expression vector used for the transformation of plant is not particularly limited as long as it can be used to express the inserted gene in a plant cell. When Agrobacterium method is used to introduce the vector into the plant, use of a binary vector such as pBI is preferable. Specific examples of binary vectors include: pBIG, pBIN19, pBI101, pBI121, and pBI221. The vector preferably includes a promoter that can cause expression of a gene in the plant. As a promoter, conventional promoters can be suitably used. Specific examples include cauliflower mosaic virus 35S promoter (CaMV35S), ubiquitin promoter, and actin promoter. Various forms of plant cell may be used, for example, such as suspension culture cells, protoplasts, leaf slices, and calluses.

The recombinant expression vector can be introduced into the plant cell by various kinds of known methods such as a polyethylene glycol method, an electroporation method, an Agrobacterium method, and a particle gun method. Reproduction of plant from transformed cells can be performed using known methods as suitably selected according to the type of plant cells.

Once a transformed plant is obtained that has incorporated a polynucleotide according to the present invention in the genome, seeds obtained from the transformed plant also include the polynucleotide. For example, from rice or other cereals transformed with a polynucleotide according to the present invention, cereals with a high tryptophan content can be obtained. The present invention also includes seeds obtained from a transformed plant.

### (6) A Screening Method and Screening Kit

A screening method according to the present invention is a method for screening for a substance that binds to either a polypeptide according to the present invention described in Section (1) above (mutant OASA2 protein), or a wild type rice anthranilate synthase (OASA2 protein), and the method includes the steps of: screening for a substance that binds to the mutant OASA2 protein; screening for a substance that binds to the wild type OASA2 protein; and comparing results of these screening steps.

A polypeptide according to the present invention, i.e., the mutant OASA2 protein, has resistance to tryptophan feedback inhibition in the biosynthetic pathway of tryptophan. It is therefore highly likely that the substance that binds to either the mutant OASA2 protein or the wild type OASA2 protein is a signaling substance involved in the feedback inhibition in the biosynthetic pathway of tryptophan. It is expected that finding such a substance with the screening method will facilitate the study of revealing the mechanism of tryptophan feedback inhibition and contribute to the development of mutant OASA protein with even stronger resistance to the feedback inhibition.

A screening kit according to the present invention is a kit for performing the screening method, and includes a wild type rice anthranilate synthase and at least one of polypeptides according to the present invention. The rest of the arrangement is not particularly limited, and the kit may additionally include reagents, instruments, and the like as suitably selected as needed. Using the transformation kit, a screening method according to the present invention can be performed both conveniently and efficiently.

The present invention is not limited to the description of the embodiments above, but may be altered by a skilled person within the scope of the claims. An embodiment based on a proper combination of technical means disclosed in different embodiments is encompassed in the technical scope of the present invention.

### [Examples]

### [Example 1: Introduction of Mutation to Rice Anthranilate Synthase Gene OASA2]

### <Insertion of Target Gene into a Cloning Vector>

Rice anthranilate synthase gene OASA2 (ACCESSION NO. AB022603) was inserted at EcoRI site in the multiple cloning site of a cloning vector pBluescript SK+ (Stratagene) to construct pBS-OASA2. The gene was inserted to give the restriction enzyme sites KpnI and SacI of the multiple cloning site in this order.

### <Mutation Introducing Primers>

When Kunkel method is used to introduce mutation into a coding gene of a target protein, a mutation-introducing oligonucleotide is prepared that is sense or antisense to the position where the mutation is introduced. When PCR is used to introduce mutation into a coding gene of a target protein, two primers, sense and antisense oligonucleotides, are prepared for the mutated position. Thus, one of these primers can be used as the mutation-introducing oligonucleotide used in the Kunkel method. Whether the mutation has been introduced or not can be confirmed by introducing a restriction enzyme site to an appropriate position that does not bring about changes in the amino acids encoded by the gene. The following describes how mutation was introduced using Kunkel method ((i) to (vi)) and PCR ((vii) and (viii)).

### <Mutation-Introducing Primers>

### (i) Substitution of asparatate residue for asparagine residue at position 351 (N351D)

A mutation-introducing primer was designed to include an amino acid substitution of asparatate residue for asparagine residue at position 351, and a restriction enzyme site (BsiWI) at a position that does not bring about changes in the encoded amino acid. The base sequence of the mutation-introducing primer is shown below. The restriction enzyme site is indicated by underline, and the small letters indicate a mutation-introducing codon.
N351D-F:
   5'-GTTTGAGAGGCGAACGTACGCCgatCCATTTGAAGTCT-3' (SEQ ID NO: 15)

The base sequence gat (asparatate, D) from position 23 to 25 of the primer N351D-F (and CGTACG (BsiWI site) from position 15 to 20) were designed from the base sequences AAT (asparagine, N) and CATACG, respectively, of wild type OASA2. The change from AAT to GAT causes a substitution of asparatate residue for asparagine residue at position 351. The change from CATACG to CGTACG introduces restriction enzyme site (BsiWI: CGTACG) without changing the amino acids (ACA and ACG both encode threonine). This makes the subsequent screening easier.

### (ii) Substitution of alanine residue for tyrosine residue at position 367 (Y367A)

Similarly, a mutation-introducing primer was designed to include an amino acid substitution of alanine residue for tyrosine residue at position 367, and a restriction enzyme site (SnaBI: TACGTA) at a position that does not bring about changes in the encoded amino acid. The base sequence of the mutation-introducing primer is shown below. The restriction enzyme site is indicated by underline, and the small letters indicate a mutation-introducing codon.
Y367A-F: 5'-GTGAACCCAAGTCCAgccATGGCATACGTACAGGCAAGAGGC-3' (SEQ ID NO: 16)

### (iii) Substitution of isoleucine residue for tyrosine residue at position 367 (Y367I)

Similarly, a mutation-introducing primer was designed to include an amino acid substitution of isoleucine residue for tyrosine residue at position 367, and a restriction enzyme site (SnaBI: TACGTA) at a position that does not bring about changes in the encoded amino acid. The base sequence of the mutation-introducing primer is shown below. The restriction enzyme site is indicated by underline, and the small letters indicate a mutation-introducing codon.
Y367I-F: 5'-GTGAACCCAAGTCCAatcATGGCATACGTACAGGCAAGAGGC-3' (SEQ ID NO: 17)

### (iv) Substitution of alanine residue for leucine residue at position 530 (L530A)

Similarly, a mutation-introducing primer was designed to include an amino acid substitution of alanine residue for leucine residue at position 530, and a restriction enzyme site (NheI: GCTAGC) at a position that does not bring about changes in the encoded amino acid. The base sequence of the mutation-introducing primer is shown below. The restriction enzyme site is indicated by underline, and the small letters indicate a mutation-introducing codon.
L530A-F:
   5'-ACGGAGACATGgctATCGCGCTAGCACTCCGCACCATT-3'(SEQ ID NO: 18)

### (v) Substitution of asparatate residue for leucine residue at position 530 (L530D)

Similarly, a mutation-introducing primer was designed to include an amino acid substitution of asparatate residue for leucine residue at position 530, and a restriction enzyme site (NheI: GCTAGC) at a position that does not bring about changes in the encoded amino acid. The base sequence of the mutation-introducing primer is shown below. The restriction enzyme site is indicated by underline, and the small letters indicate a mutation-introducing codon.
L530D-F:
   5'-ACGGAGACATGgacATCGCGCTAGCACTCCGCACCATT-3' (SEQ ID NO: 19)

### (vi) Substitution of tyrosine residue for glycin residue at position 522 and asparatate residue for leucine residue at position 530 (G522Y + L530D)

Similarly, a mutation-introducing primer was designed to include amino acid substitutions of glycin residue for tyrosine residue at position 522 and asparatate residue for leucine residue at position 530, and a restriction enzyme site (BciVI: GTATCC/GGATAC) at a position that does not bring about changes in the encoded amino acid. The base sequence of the mutation-introducing primer is shown below. The restriction enzyme site is indicated by underline, and the small letters indicate a mutation-introducing codon.
G522Y+L530D-F:
   5'-AGTGGCGGCCTTGGAtacATATCATTTGACGGAGACATGgatATCGCTCTTG-CACT-3' (SEQ ID NO: 20)

### (vii) Substitution of phenylalanine residue for serine residue at position 126 (S126F)

Mutation-introducing primers were designed to include an amino acid substitution of phenylalanine residue for serine residue at position 126. PCR was used to introduce mutation and as such sense and antisense oligonucleotides were prepared. The base sequences of the mutation-introducing primers are shown below. The small letters indicate mutation-introducing codons.
S126F-F:
   5'-GCTTCCTCTTCGAGttcGTCGAGCAGGGGCC-3' (SEQ ID NO: 37)
S126F-R:
   5'-GGCCCCTGCTCGACgaaCTCGAAGAGGAAGC-3' (SEQ ID NO: 38)

The base sequence ttc (phenylalanine, F) from position 15 to 17 of the primer S126F-F was designed from TCC (serine, S) of wild type OASA2. The change from TCC to TTC causes a substitution of phenylalanine residue for serine residue at position 126. Primer S126F-R is complementary to S126F-F.

### (viii) Substitution of leucine residue for alanine residue at position 369 (A369L)

Similarly, mutation-introducing primers were designed to include an amino acid substitution of leucine residue for alanine residue at position 369. PCR was used to introduce mutation and as such sense and antisense oligonucleotides were prepared. The base sequences of the mutation-introducing primers are shown below. The small letters indicate mutation-introducing codons.
A369L-F:
   5'-CAAGTCCATACATGctaTATGTACAGGCAA-3' (SEQ ID NO: 39)
A369L-R:
   5'-TTGCCTGTACATAtagCATGTATGGACTTG-3' (SEQ ID NO: 40)

primer A369L-R is complementary to primer A369L-F.

### <Preparation of Mutation-Introduced DNA by Kunkel Method>

Kunkel method was performed according to procedures described in the following References 1 to 3.

Reference 1: Kunkel, T. A. (1985) Rapid and efficient site-specific mutagenesis without phenotypic selection. Proceedings of the National Academy of Science of the USA, 82: 488-492

Reference 2: Kunkel, T. A., Bebenek, K. and McClary, J. (1991) Efficient site-directed mutagenesis using uracil-containing DNA. Methods in Enzymology, 204: 125-139

Reference 3: "Molecular Cloning (Third Edition)" (J. Sambrook & D. W. Russell, Cold Spring Harbor Laboratory Press, (2001) Chapter 13

For example, for the substitution of asparatate residue for asparagine residue at position 351, the mutation-introducing primer of (i) was used to introduce mutation by the Kunkel method, using the pBS-OASA2 vector as a template. The same template was used to introduce mutation with the primers of (ii), (iv), (v), and (vi). By these procedures, mutant DNAs N351D, Y367A, L530A, L530D, and G522Y+L530D were prepared.

The double-mutant DNA N351D+L530A was prepared by repeating the mutation-introducing procedure twice. Specifically, using the pBS-OASA2 vector as a template, N351D mutant DNA was prepared first with the mutation-introducing primer of (i). Then, mutant DNA with two mutations (N351D+L530A) was prepared with the mutation-introduced primer of (iv), using N351D mutant plasmid DNA (pBS-OASA2 (N351D) vector) as a template. In the same manner, mutant DNA with two mutations (N351D+L530D) was obtained with the mutation-introducing primer of (v), using N351D mutant plasmid DNA (pBS-OASA2 (N351D) vector) as a template. Similarly, mutant DNA with two mutations (Y367A+L530D) was obtained with the mutation-introducing primer of (v), using Y367A mutant plasmid DNA (pBS-OASA2 (Y367A) vector) as a template.

Triple-mutant DNA was prepared by Kunkel method, using plasmid DNA with the double-mutant DNA as a template. Specifically, mutant DNA with three mutations (N351D+Y367A+L530A) was obtained with the mutation-introducing primer of (ii), using plasmid DNA (pBS-OASA2 (N351D+L530A) vector) with the mutations N351D+L530A as a template. In the same manner, mutant DNA with three mutations (Y367A+G522Y+L530A) was obtained with the mutation-introducing primer of (ii), using plasmid DNA (pBS-OASA2 (G522Y+L530D) vector with the mutations G522Y+L530D as a template. Similarly, mutant DNA with three mutations (Y367I+G522Y+L530A) was obtained with the mutation-introducing primer of (iii), using plasmid DNA (pBS-OASA2 (G522Y+L530D) vector) with the mutations G522Y+L530D as a template.

### <Preparation of Mutation-Introduced DNA by RCR Method>

Mutagenesis by a PCR method was performed according to procedures described in the following publication.

Higuchi R, Krummel B, Saiki RK (1988) A general method of in vitro preparation and specific mutagenesis of DNA fragments: study of protein and DNA interactions. Nucleic Acids Res 16: 7351-7367 PCR was run separately for each region on the 5' end and 3' end to create a redundant region for the OASA2 gene in the primer regions. For example, when the mutation-introducing primers of (vii) were used that cause a substitution of phenylalanine for serine at position 126, PCR for the 5' end was run with a combination of cloning sense primer (5'-AAAACTAGTATGGAGTCCATCGCCGCCGCCACG-3': SEQ ID NO: 41, underline indicates restriction enzyme site SpeI) and primer S126F-R, using pBS-OASA2 vector as a template. For the 3' end, PCR was run with a combination of primer S126F-F and cloning antisense primer (5'-AAAGTCGACTGAGAGAGACTCTATTCCTTGTC-3': SEQ ID NO: 42, underline indicates restriction enzyme site SalI), using pBS-OASA2 vector as a template. For the mutation-introducing primers of (viii), PCR was run by combining the primers in the same manner and using the same templates.

For preparation of double-mutant DNA S126F+L530D, plasmid DNA was used as a template that had been prepared by introducing a mutation, that causes an amino acid substitution of asparatate residue for leucine residue at position 530 of OASA2, into the pBS-OASA2 vector using the Kunkel method. Thus, by the PCR using the mutation-introducing primers (S126F-F and S126F-R) of (vii), a mutant gene can be obtained that has incorporated two mutations in OASA2: a substitution of phenylalanine residue for serine residue at position 126, and a substitution of asparatate residue for leucine residue at position 530. The primers were used in combinations as described above. Double-mutation DNA A369L+L530D is also prepared by PCR using the same kind of templates and the mutation-introducing primers (A369L-F and A369L-R), which yields a mutant gene with two mutations in OASA2: a substitution of leucine residue for alanine residue at position 369, and a substitution of asparatate residue for leucine residue at position 530.

PCR reaction was run with the following components: 1 × Pyrobest buffer II (TaKaRa), 0.2 mM dNTP, 0.5 µM sense and antisense primers, 0.025 units/µl Pyrobest DNA polymerase (TaKaRa), and 10 ng template DNA. Using a PCR reactor (Takara Shuzo Co., Ltd., TaKaRa PCR Thermal Cycler MP TP3000), the PCR reaction was performed with the following cycling parameters: one cycle consisting of retention for 2 minutes at 95°C; 25 cycles consisting of 98°C for 20 seconds, 60°C for 35 seconds, and 72°C for 3 minutes; and one cycle consisting of retention for 10 minutes at 72°C. This was followed by cooling at 4°C.

The amplified PCR product (fragments of 5' region and fragments of 3' region) was diluted 20 times, and 1 µl of the diluted solution was added to each PCR reaction solution [1×Pyrobest buffer II (TaKaRa), 0.2 mM dNTP, 0.025 units/µl Pyrobest DNA polymerase (TaKaRa)]. Using the PCR reactor, the extension reaction was performed according to the following cycling parameters: one cycle consisting of retention for 2 minutes at 95°C; and 5 cycles consisting of 98°C for 20 seconds, 60°C for 35 seconds, and 72°C for 3 minutes. The reaction synthesized DNA fragments that were joined together from the cloning sense primer to the cloning antisense primer.

Immediately after the reaction, the cloning sense primer and the cloning antisense primer were added to the concentration of 0.2 µM for each primer. Then, using the PCR reactor, the PCR reaction was performed according to the following cycling parameters: one cycle consisting of retention for 2 minutes at 95°C; 20 cycles consisting of 98°C for 20 seconds, 60°C for 35 seconds, and 72°C for 3 minutes; and one cycle consisting of retention for 10 minutes at 72°C. This was followed by cooling at 4°C.

The reaction amplified the DNA fragments that were joined together from the cloning sense primer to the cloning antisense primer, and the DNA fragments were inserted at SpeI site and SalI site in multiple cloning site of cloning vector pBluescript KS+ (Stratagene) to construct pBS-OASA2(S126F), pBS-OASA2(A369L), pBS-OASA2 (S126F/L530D), and pBS-OASA2(A369L/L530D).

### [Example 2: Synthesis of Protein by Wheat Embryo Acellular System]

### (1) Synthesis of Transcription Template DNA by Split-PCR Method

### <Preparation of Template for Split-PCR>

It is known that OASA2 gene resides in the nuclear genome of rice, and that the synthesized protein moves into the chloroplast where it exhibits its action. The N terminus region of the synthesized protein has a signal sequence, which is removed to turn the protein into a mature enzyme and allows it to exhibit its action. Considering this, for the synthesis of OASA2 protein as a mature enzyme in a wheat embryo acellullar synthesis system, a primer was designed such that the synthesized protein did not include the signal sequence of 49 residues at the N terminus region. More specifically, ATG start codon was placed downstream of the linker sequence that enables Split-PCR in the wheat embryo acellular system, and a primer with a total length of 36mer were designed that had the base sequence from position 148 to 164 of the OASA2 gene. Further, two kinds of antisense primers for Split-PCR were prepared for the vector (pBluescript SK+) that had incorporated the OASA2 gene. The antisense primers were set in positions on the complementary strand of the sense Split-PCR primer. These antisense primers were designated as antisense Split-PCR primer 1 and antisense Split-PCR primer 2, respectively, in this order from the far side of the inserted DNA. The base sequences of the Split-PCR primers are as follows. Split-PCR will be described later.
Sense Split-PCR primer:
   5'-CCTCTTCCAGGGCCCAATGTGCTCCGCGGGGAAGCC-3' (SEQ ID NO: 21, underline indicates the linker sequence)
Antisense Split-PCR primer 1:
   5'-GGAGAAAGGCGGACAGGTAT-3' (SEQ ID NO: 22)
Antisense Split-PCR primer 2:
   5'-GGGGAAACGCCTGGTATCTT-3' (SEQ ID NO: 23)

As a template, the pBluescript SK+ vector that has incorporated the mutated OASA2 gene by the Kunkel method is used, and PCR or other amplification reactions are performed with the sense Split-PCR primer and the antisense Split-PCR primer 1. The amplified DNA fragments can then be used as a template for the next round of Split-PCR.

The PCR reaction solution had the following components:
1 × Pyrobest buffer II (TaKaRa), 0.2 mM dNTP, 0.5 µM sense Split-PCR primer, 0.5 µM antisense Split-PCR primer 1, 0.025 units/µl Pyrobest DNA polymerase (TaKaRa), and 10 ng template DNA (pBS-OASA2 vector with the mutation). Using a PCR reactor (Takara Shuzo Co., Ltd., TaKaRa PCR Thermal Cycler MP TP3000), the PCR reaction was performed with the following cycling parameters: one cycle consisting of retention for 2 minutes at 95°C; 25 cycles consisting of 98°C for 20 seconds, 60°C for 35 seconds, and 72°C for 3 minutes; and one cycle consisting of retention for 10 minutes at 72°C. This was followed by cooling at 4°C. The reaction synthesized DNA fragments that were joined together from the sense Split-PCR primer to the antisense Split-PCR primer 1. The amplified DNA was then used as a template for the next round of Split-PCR.

### <Synthesis of Transcriptional Template DNA >

In order to use the Split-PCR template DNA as the template DNA of transcription, a promoter of SP6 RNA polymerase and a translation enhancer sequence (omega sequence) that originates in tobacco mosaic virus (TMV) need to be attached upstream of the 5' end of the fragment. For this purpose, Split-PCR was performed according to the method of Sawasaki et al. (Sawasaki et al. (2002) Proc Natl Acad Sci U S A 99, 14652-14657) Specifically, the Split-PCR template DNA was diluted 50 times, and 1 µl of the diluted solution was added to PCR reaction solution [1 × EX Taq buffer (TaKaRa), 0.2 mM dNTP, 0.025 units/µl TaKaRa EX Taq (TaKaRa), 0.2 µM SP6 promoter primer, 1nM TMV-Omega primers, and 0.2 µM antisense Split-PCR primer 2]. Using the TaKaRa PCR reactor, the PCR reaction was performed with the following cycling parameters: one cycle consisting of retention for 2 minutes at 95°C; 35 cycles consisting of 98°C for 20 seconds, 60°C for 35 seconds, and 72°C for 3 minutes; and one cycle consisting of retention for 10 minutes at 72°C. This was followed by cooling at 4°C. The base sequences of the SP6 promoter primer and TMV-Omega primer are shown below. Underline indicates SP6 promoter sequence, and the small letters indicate a redundant sequence of the primers.
SP6 promoter primer:
   5'-GCGTAGCATTTAggtgacact-3' (SEQ ID NO: 24)
TMV-Omega primer:
   5'-ggtgacactATAGAAGTATTTTTACAACAATTACCAACAACAACAACAAACAAC AACAACATTACATTTTACATTCTACAACTACCTCTTCCAGGGCCCAATG-3' (SEQ ID NO: 25)

As in the SP6 promoter primer and TMV-Omega primer, primers that are split into upstream and downstream primers with a partially redundant sequence between the 3' end of the upstream primer and the 5' end of the downstream primer will be referred to as split primers. PCR reaction using such primers will be referred to as Split-PCR.

### (2) Synthesis of mRNA for the Wheat Embryo Acellular System

The PCR product obtained in Section (1) above was directly used as a template to synthesize (transcribe) mRNA. Specifically, 1/10 the amount of PCR product obtained in Section (1) was added to a transcriptional reaction solution [80 mM HEPES-KOH (pH7.8), 16 mM Mg(OAc)₂, 10 mM spermidine, 10 mM DTT, 3 mM NTP, 1 unit/µl RNasin RNase inhibitor (Promega), and 1 unit/µl SP6 RNA polymerase (Promega)]. After 2 hours of reaction at 37°C, the reaction mixture was precipitated with ethanol and the precipitates were washed with 70% ethanol. The precipitates were then dissolved in an appropriate amount of sterilized water, and the quantity of RNA was calculated by measuring absorbance at 260 nm.

### (3) Synthesis of Protein by the Wheat Embryo Acellular System

The mRNA synthesized in Section (3) above was used as a template, and protein was synthesized according to the method of Sawasaki et al. (Sawasaki et al. (2002) Proc Natl Acad Sci U S A 99, 14652-14657) Specifically, about 30 to 35 µg of mRNA was added to a dialysis cup containing 50 µl of wheat embryo acellular protein reaction mixture, and the dialysis cup was dipped into each well of a 24-well plate containing 1ml of substrate solution per well. The solution was incubated for 24 hours at 26°C.

### [Example 3: Activity Measurement of Mutant OASA2 Protein]

### (1) Quantification of OASA2 Protein by Western Blot Method

The OASA2 protein synthesized by the wheat embryo acellular system was quantified using rabbit anti-OASA2 antibody that had been prepared based on the peptide fragment with the sequence at position 161 to 175 (MDHEKGKVTEQVVDD) of the amino acid sequence of OASA2 protein. A refined sample of OASA2 protein was also used for the quantification. Western blot analysis was performed according to the method of Towbin et al. (Towbin, H., Staehelin, T. and Gordon, J. 1979. Electrophoretic transfer of proteins from polyacrylamide gels to nitrocellulose sheets: procedure and some applications. Proc Natl Acad Sci USA 76: 4350-4354). The estimated quantity of OASA2 protein was used for the correction of enzyme activity, as will be described later.

### (2) Activity Measurement of OASA2 Protein

The OASA2 protein, which is the α subunit of rice anthranilate synthase catalyzes the reaction producing anthranilate from chorismate, using ammonia that originates in the amide group of glutamine supplied by the β subunit. In the organism, the OASA2 protein exhibits its activity to synthesize anthranilate (hereinafter, referred to as "AS activity") using ammonia supplied by the β subunit. The OASA2 protein can also exhibit its enzyme activity in response to externally supplied ammonia, for example, such as NH₄Cl.

As is known, activity of OASA2 protein is under the feedback inhibition by tryptophan. It is therefore necessary to eliminate tryptophan in the protein synthesis reaction mixture, and replace the environment of the expressed protein with a buffer component for AS activity measurement. To this end, the protein synthesis reaction mixture was replaced with buffer A (50 mM Tris-HCl, pH7.6; 0.05 mM EDTA; 2 mM MgCl₂; 0.05 mM DTT; 5% glycerol), using Microspin G-25 columns (Amersham Biosciences)

### <Enzyme Activity Measurement 1>

Ninety µl of reaction solution (20 mM Tris-HCl, pH 8.3; 100 mM NH₄Cl; 0.5mM chorismate; 10 mM MgCl₂) was supplemented with 2.5 µl of crude extract exchanged with buffer, and the reaction mixture was allowed to react for 1 hour at 32°C. The reaction was stopped by adding 10 µl of 1N HCl, and synthesized anthranilate was extracted with 300 µl of ethyl acetate. The yield of anthranilate was measured with Wallac 1420 ARVOsx-FL (Perkin-Elmer Life Sciences Japan. Co., Ltd.) at the excitation wavelength of 355 nm/fluorescence (emission) 460 nm. Here, for the analysis of activity under feedback inhibition by tryptophan, tryptophan was added to a final concentration of 10 µM or 100 µM.

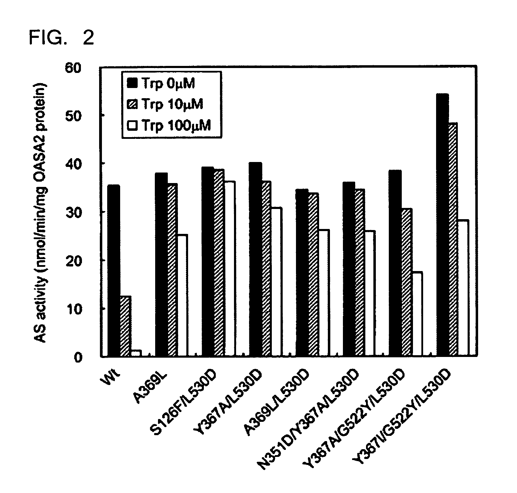

The results are shown in Figure 1 and Figure 2. As clearly shown in Figure 1, the six kinds of mutant OASA2 proteins (N351D, L530A, L530D, N351D/L530A, N351D/L530D, and G522Y/L530D) had improved enzyme activity over the wild type (wt). Further, as clearly shown in Figure 2, without tryptophan, the seven kinds of mutant OASA2 proteins (A369L, S126F/L530D, Y367A/L530D, A369L/L530D, N351D/Y367A/L530D, Y367A/G522Y/L530D, and Y3671/G522Y/L530D) had enzyme activity substantially matching or exceeding enzyme activity of the wild type, and had acquired resistance to tryptophan feedback inhibition.

### <Enzyme Activity Measurement 2>

In order to more closely access enzyme characteristics of the five kinds of OASA2 proteins that had acquired resistance to tryptophan feedback inhibition, enzyme activity and tryptophan feedback inhibition were analyzed in a reaction system using the β subunit of rice anthranilate synthase.

Ninety µl of reaction solution (20 mM Tris-HCl, pH 8.3; 5 mM glutamine; 0.2 to 0.8 mM chorismate; 10 mM MgCl₂) was supplemented with 2.5 µl of crude extract exchanged with buffer, and the reaction mixture was allowed to react for 1 hour at 32°C. The preparation of rice anthranilate synthase β subunit and quantification of the product by the Western blot method were performed according to the method of Kanno et al. (Kanno et al.(2004) Plant Molecular Biology 54,11-22) The reaction was stopped by adding 10 µl of 1N HCl, and synthesized anthranilate was extracted with 300 µl of ethyl acetate. The yield of anthranilate was measured with Wallac 1420 ARVOsx-FL (Perkin-Elmer Life Sciences Japan. Co., Ltd.) at the excitation wavelength of 355 nm/fluorescence (emission) 460 nm. Here, for the analysis of activity under feedback inhibition by tryptophan, tryptophan was added to a final concentration of 10 µM, 25 µM, or 50 µM.

The results are shown in Table 1 and Figure 3. As clearly shown in Table 1, the nine kinds of mutant OASA2 proteins (S126F, Y367A, A369L, S126F/L530D, Y367A/L530D, A369L/L530D, N351D/Y367A/L530D, Y367A/G522Y/L530D, and Y367I/G522Y/L530D) had enzyme activity substantially matching or exceeding enzyme activity of the wild type (wt), except for S126F and Y367A. More specifically, compared with the wild type (wt), the enzyme activity increased about 1 fold in A369L, about 2.5 fold in S126F/L530D, about 2.2 fold in Y367A/L530D, about 1.8 fold in A369L/L530D, about 1.3 fold in Y367A/G522Y/L530D, about 1.2 fold in N351D/Y367A/L530D, and about 0.8 fold in Y367I/G522Y/L530D.

**[Table 1]**

| OASA2 Mutant | Vₘₐₓ (nmol/min/mg OASA2 Protein) | Relative Ratio (fold) |
|---|---|---|
| Wt | 215 | 1.00 |
| S126F | 103 | 0.48 |
| Y367A | 84 | 0.39 |
| A369L | 206 | 0.96 |
| S126F/L530D | 546 | 2.54 |
| Y367A/L530D | 467 | 2.17 |
| A369L/L530D | 385 | 1.79 |
| N351D/Y367A/L530D | 251 | 1.17 |
| Y367A/G522Y/L530D | 277 | 1.29 |
| Y367I/G522Y/L530D | 168 | 0.78 |

Figure 3 represents enzyme activity of the wild type and mutant OASA2 proteins at varying concentrations of tryptophan, relative to 100% enzyme activity without tryptophan. As clearly shown in Figure 3, the enzyme activity of the wild type falls below 10% of the enzyme activity without tryptophan when the amount of supplemented tryptophan is 50 µM or greater. In contrast, the nine kinds of mutant OASA2 proteins (S126F, Y367A, A369L, S126F/L530D, Y367A/L530D, A369L/L530D, N351D/Y367A/L530D, Y367A/G522Y/L530D, and Y367I/G522Y/L530D) maintained 30% or greater enzyme activity at the tryptophan concentration of 50 µM.

### [Example 4: Analysis of Free Tryptophan Content in Yeast TRP2 Gene Defective Mutant Strains (MATalpha his3Δ1 leu2Δ0 met15Δ0 ura3Δ0 trp2::KANMX)Expressing Mutant OASA2 Gene]

Yeasts with a defective TRP2 gene, analogous to OASA2 gene, require tryptophan for growth. It can therefore be said that TRP2 activity is complemented by OASA2 gene if OASA2 gene introduced into the yeast allows for growth without tryptophan. Further, the activity of mutant OASA2 gene can be assayed by causing the yeast to express the mutant OASA2 gene having resistance to tryptophan feedback inhibition and by measuring accumulation of free tryptophan in the organism.

Expression vectors were constructed by performing PCR with the primers below. As the templates, pBluescript vectors were used that had incorporated therein mutant OASA2 genes having resistance to tryptophan feedback inhibition (S126F, Y367A, A369L, S126F/L530D, Y367A/L530D, A369L/L530D, N351D/Y367A/L530D, Y367A/G522Y/L530D, Y3671/G522Y/L530D) and wild type (Wt) OASA2 gene. As described above, the N terminus region of the OASA2 protein includes a chloroplast transit signal, and as such the primers were designed to exclude the signal sequence in the product of expression, as in the Split-PCR primers described in Section (2) above. The sense primer and antisense primer had restriction enzyme sites KpnI site (GGTACC) and EcoRI site (GAATTC), respectively, so that these primers could be inserted at the restriction enzyme KpnI/EcoRI sites of yeast expression vector pYES2 (Invitrogen). The restriction enzyme sites are indicated by underline. The pYES2 vector has URA3 gene, enabling induction of protein expression to be controlled by galactose.
Sense primer:
   5'-AAAGGTACCATGTGCTCCGCGGGGAAGCC-3' (SEQ ID NO: 27)
Antisense primer:
   5'-AAAGAATTCTGAGAGAGACTCTATTCCTTGTC-3' (SEQ ID NO: 28)

The PCR reaction solution had the following components:
1 × Pyrobest buffer II (TaKaRa), 0.2 mM dNTP, 0.5 µM sense primer, 0.5 µM antisense primer 1, 0.025 units/µl Pyrobest DNA polymerase (TaKaRa), and 10 ng template DNA (pBS-OASA2 vector with the mutation). Using a PCR reactor (Takara Shuzo Co., Ltd., TaKaRa PCR Thermal Cycler MP TP3000), the PCR reaction was performed with the following cycling parameters: one cycle consisting of retention for 2 minutes at 95°C; 25 cycles consisting of 98°C for 20 seconds, 60°C for 35 seconds, and 72°C for 3 minutes; and one cycle consisting of retention for 10 minutes at 72°C. This was followed by cooling at 4°C.

Cloning of DNA fragments with the vector was performed according to the method of Sambrook et al. (Molecular Cloning (Third Edition) (J. Sambrook & D. W. Russell, Cold Spring Harbor Laboratory Press, 2001)). Basic procedures, including transformation of the constructed vector into yeast TRP2 gene defective strain followed the method of Kaiser et al. (Methods in Yeast Genetics: A Cold Spring Harbor Laboratory Course Manual (Chris Kaiser, Susan Michaelis, Aaron Mitchell, Cold Spring Harbor Laboratory, 1994)).

The yeast transformant was separated into single colonies on synthetic complete agar medium (0.67% amino acid-less bactoyeast nitrogen base, uracil-free 0.2% dropout mixture, 2% bactoagar) supplemented with 2% glucose. The cells were then streaked onto synthetic complete agar medium (0.67% amino acid-less bactoyeast nitrogen base, uracil- and tryptophan-less 0.2% dropout mixture, 2% bactoagar) supplemented with 2% galactose, and incubated at 30°C for 2 days. Twenty mg of cells from the agar medium was suspended in 105 µl of distilled water and were processed at 100°C for 20 minutes. Then, a 595 µl mixture of chloroform and methanol (5:12, v/v) was added, and the mixture, after thorough agitation, was centrifuged at 20,000 × g for 10 minutes. The supernatant was transferred into a new tube and supplemented with 175 µl of distilled water and 263 µl of chloroform. The mixture was vigorously agitated, and centrifuged at 20,000 × g for 10 minutes. The extracted aqueous layer was transferred into a new tube, and was evaporated under reduced pressure. This was dissolved in 200 µl of 10 mM NaOH to prepare a tryptophan extractant. The preparation was then applied to a high-performance liquid chromatography (HPLC) device (the product of Waters, Waters Alliance HPLC FLD System 2695) and free tryptophan content was measured. For HPLC, a Xterra RP18 column (4.6 × 150 mm) (Waters) was used, and detection of tryptophan was made at the excitation wavelength 278nm/fluorescence wavelength 348 nm.

The results are shown in Table 2 below. As clearly shown in Table 2, the concentration of accumulated free tryptophan was greater in yeasts that had expressed the mutant OASA2 gene having resistance to tryptophan feedback inhibition (S126F, Y367A, A369L, S126F/L530D, Y367A/L530D, A369L/L530D, N351D/Y367A/L530D, Y367A/G522Y/L530D, Y367I/G522Y/L530D) compared with yeast that had expressed wild type (Wt) OASA2 gene (1.9 to 2.3 fold increase).

**[Table 2]**

| Measured Yeast Transformant | Free Tryptophan Content (pmol/mg wet weight) | Relative Amount (fold) |
|---|---|---|
| Wt | 126 ± 7 | 1.0 |
| S126F | 197 ± 3 | 1.6 |
| Y367A | 267 ± 5 | 2.1 |
| A369L | 243 ± 10 | 1.9 |
| S126F/L530D | 266 ± 6 | 2.1 |
| Y367A/L530D | 284 ± 5 | 2.3 |
| A369L/L530D | 255 ± 6 | 2.0 |
| N351D/Y367A/L530D | 295 ± 2 | 2.3 |
| Y367A/G522Y/L530D | 251 ± 17 | 2.0 |
| Y367I/G522Y/L530D | 244 ± 9 | 1.9 |

### [Example 5: Preparation of Rice Callus Transformant Expressing Mutant OASA2 Gene and Analysis of Free Tryptophan Content Therein]

### <Construction of Recombinant Vector for Introducing Exogenous Gene>

Construction of recombinant vectors for introducing exogenous genes was performed according to the methods described in the following references:
Reference 1: Urushibara S, Tozawa Y, Kawagishi-Kobayashi M and Wakasa K (2001) Efficient transformation of suspension-cultured rice cells mediated by Agrobacterium tumefaciens. Breeding Science 51: 33-38
Reference 2: Tozawa Y, Hasegawa H, Terakawa T and Wakasa K (2001) Characterization of rice anthranilate synthase alfa subunit genes OSASA1 and OSASA2: tryptophan accumulation in transgenic rice expressing a feedback-insensitive mutant of OASA1. Plant Physiology 126: 1493-1506
Reference 3: Hiei Y, Ohta S, Komari T and Kumashiro T (1994) Efficient transformation of rice (Oryza sativa) mediated by Agrobacterium and sequence analysis of boundaries of the T-DNA. Plant Journal 6: 271-282
Exogenous gene-introducing recombinant vector pUB-Hm (Urushibara et al., Breeding Science 51: 33-38 (2001)) includes a corn ubiquitin promoter, a first intron, a restriction enzyme Sse8387I site, a NOS terminator, and a hygromycin-resistant gene. A recombinant vector for transforming rice can be constructed by inserting a target exogenous gene at the Sse8387I site.

Full-length wild type (Wt) OASA2 gene was amplified by PCR using the sense and antisense primers below. As the template, pBS-OASA2 described in Example 1 was used. The sense primer and antisense primer had restriction enzyme sites SpeI site (ACTAGT) and SalI site (GTCGAC), respectively, which are indicated below by underline.
Sense primer:
   5'-AAAACTAGTATGGAGTCCATCGCCGCCGCCACG-3' (SEQ ID NO: 43)
Antisense primer:
   5'-AAAGTCGACTGAGAGAGACTCTATTCCTTGTC-3' (SEQ ID NO: 44)

For full-length mutant OASA2 genes (Y367A, Y367A/L530D, S126F/L530D), DNA fragments were prepared according to the procedure described in <Preparation of Mutation-Introduced DNA by RCR Method> in Example 1, using the sense primer (SEQ ID NO: 43) and the antisense primer (SEQ ID NO: 44). As the template, pBS-OASA2 was used. The DNA fragments amplified by PCR were digested with restriction enzymes SpeI and SalI, and the resulting DNA fragments (1) were inserted into SpeI/SaII sites of expression vector pEU3s for the wheat embryo acellular synthesis system, the expression vector pEU3s being a modification of expression vector pEU3b for the wheat embryo acellular synthesis system, constructed by Sawasaki et al. (Sawasaki et al. (2002) Proc Natl Acad Sci U S A 99, 14652-14657), in which Sse8387I sites are inserted at the both ends of SpeI/SalI sites in the multiple cloning site.

Separately, pEU3s plasmid vector including the mutant OASA2 gene (Y367A, Y367A/L530D, S126F/L530D) or wild type (Wt) OASA2 gene was digested with restriction enzyme Sse8387I to obtain a 1.8 kb DNA fragment (2).

The pUB-Hm plasmid vector digested with the restriction enzyme Sse8387I, and the DNA fragment (2) were treated with a DNA ligation kit ver.2 (TAKARA BIO INC.) to perform a DNA ligation reaction. As a result, a circular recombinant vector was constructed. The recombinant vector had a first intron region downstream of the ubiquitin promoter region; mutant OASA2 gene (Y367A, Y367A/L530D, S126F/L530D) or wild type (Wt) OASA2 gene inserted and ligated between the first intron region and a NOS terminator region; and a hygromycin-resistant gene expressed in plants. The resulting recombinant vectors for introducing exogenous genes were designated as pUB-OASA2(wt)-Hm (including wild type OASA2 gene), pUB-OASA2(Y367A)-Hm (including mutant OASA2 gene (Y367A)), pUB-OASA2(Y367A/L530D)-Hm (including mutant OASA2 gene (Y367A/L530D)), and pUB-OASA2(S126F/L530D)-Hm (including mutant OASA2 gene (S126F/L530D)).

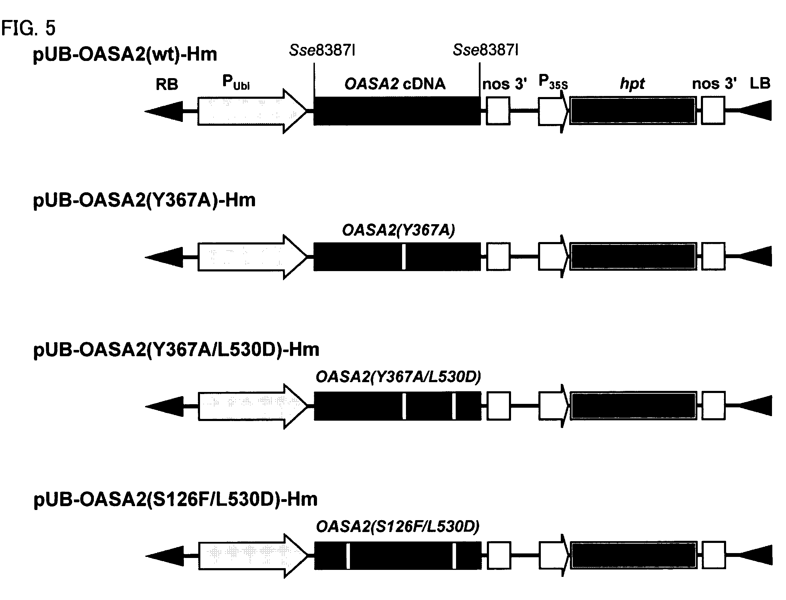

Figure 5 schematizes pUB-OASA2(wt)-Hm, pUB-OASA2(Y367A)-Hm, pUB-OASA2(Y367A/L530D)-Hm, and pUB-OASA2(S126F/L530D)-Hm. In Figure 5, PUbi indicates a ubiquitin promoter region, P35S indicates a cauliflower mosaic virus 35S promoter region, nos3' indicates a NOS terminator region, hpt indicates a hygromycin-resistant gene region, Sse8387I indicates a restriction enzyme site, RB indicates a right border sequence (Right Border), and LB indicates a left border sequence (Left Border).

### <Preparation of Agrobacterium>

Fifty ml of YEB medium (bactopeptone 5 g/l, bactobeef extract 5 g/l, bactoyeast extract 1 g/l, sucrose 5 g/l, 2 mM MgCl₂, pH 7) was inoculated with Agrobacterium (*Agrobacterium tumefaciens* EHA101). After 16 hours of shaking incubation at 30°C, the cell culture was centrifuged at 4°C to obtain cell precipitates. The cell precipitates were suspended in ice-cooled 10mM Tris-HCl (pH 7.5) and were centrifuged to obtain precipitates, which were then suspended in 0.5 ml of YEB medium. Each 5 µg solution of the three kinds of exogenous gene introducing recombinant vectors was added to 0.2 ml of the cell suspension and thoroughly mixed therein. The solution was immediately frozen and melted at 37°C. This was repeated a total of thee times. The suspension was then applied to 16 ml of YEB medium, and the cells were incubated with agitation at 30°C for 2 hours. The cell culture was applied to an agar medium that had been prepared by supplementing L medium (bactotryptone 10 g/l, bactoyeast extract 5 g/l, NaCl 5g/l, bactoagar 15 g/l, pH 7.5) with 100 mg/l of kanamycin and 50 mg/l of hygromycin. The cells were incubated at 30°C for 36 hours, and the resulting colonies were obtained as transformed Agrobacterium that had incorporated the recombinant vector.

### <Preparation of Rice Callus>

The mature seeds of rice (variety: Nipponbare) were threshed. The seeds with hulls were sterilized by being placed in a 70% ethanol solution for 60 seconds, and then in a solution of sodium hypochlorite (about 4% available chlorine) for 6 minutes. This was followed by washing with sterilized water.

The sterilized rice seeds were inoculated on 2N6 solid medium (Urushibara et al., Breeding Science 51: 33-38 (2001)) that had been prepared by supplementing an inorganic salt component of N6 medium with sucrose 30 g/l, 2,4-D 2 mg/l, casamino acid 1 g/l, and Gelrite 2g/l, and N6 vitamin. Calluses were formed after 3-week incubation at 28°C. The calluses were then excised from the albumen portion, and were transferred onto a medium of the same components, where the calluses were incubated for 7 days.

### <Transformation of Rice Callus>

The transformed Agrobacterium for introducing genes was suspended in 30 ml of liquid medium that had been prepared by supplementing the components of AA medium (Hiei et al., Plant Journal 6: 271-282 (1994)) with sucrose 20 g/l, 2,4-D 2 mg/l, kinetin 0.2 mg/l, and acetosyringone 10 mg/l. The cell suspension so obtained was placed in a 9 cm Petri dish, and 100 rice calluses were immersed therein for 5 minutes. After immersion, excess moisture on calluses was removed with a paper towel, and the calluses were inoculated, 20 each, on a Petri dish containing 2N6CO solid medium that had been prepared by supplementing an inorganic salt component of N6 medium with sucrose 30 g/l, glucose 10 g/l, 2,4-D 2 mg/l, casamino acid 1 g/l, Gelrite 2 g/l, and acetosyringone 10 mg/l. The calluses were incubated in dark at 24°C for 3 days to infect the rice calluses with Agrobacterium.

### <Screening of Calluses>

The transformed calluses that had incorporated the recombinant vector were washed with sterilized water supplemented with 500 mg/l carbenicillin, so as to remove Agrobacterium. After removing excess moisture, the calluses were transferred, 20 each, to a Petri dish containing 2N6SE solid medium that had been prepared by supplementing an inorganic salt component of N6 medium with sucrose 30 g/l, 2,4-D 2 mg/l, carbenicillin 500 mg/l, hygromycin 30 mg/l, casamino acid 1 g/l, and Gelrite 2 mg/l. The calluses were incubated in dark at 28°C for 3 weeks to obtain hygromycin-resistant transformed calluses. The proliferating calluses were transferred onto a medium of the same components, and the hygromycin-resistant transformed calluses were incubated at 28°C for 3 weeks.

After 3 weeks, DNA was extracted from part of the calluses, and the DNA was identified as being of transformed calluses by PCR using the sense and antisense primers shown below.
Sense primer:
   5'-GGATGGCACCCGCAGCAGATCG-3' (SEQ ID NO: 45)
Antisense primer:
   5'-GTACTCATCACTTGTCATGGTTG-3' (SEQ ID NO: 46)

The presence of OASA2 mutant gene in the transformant was confirmed by amplification of a fragment, corresponding to 402 base pairs, originating in the transforming vector gene. Because the OASA2 gene originally contained in the genome has intron sequences whereas the transformed gene does not, the DNA amplified product of 402 base pairs originates in only the transforming gene.

The PCR reaction solution had the following components:
1 × Pyrobest buffer II (TaKaRa), 0.2 mM dNTP, 0.5 µM sense primer (SEQ ID NO: 45), 0.5 µM antisense primer (SEQ ID NO: 46), 0.025 units/µl Pyrobest DNA polymerase (TaKaRa), and 10 ng template DNA (callus DNA). Using a PCR reactor (Takara Shuzo Co., Ltd., TaKaRa PCR Thermal Cycler MP TP3000), the PCR reaction was performed with the following cycling parameters: one cycle consisting of retention for 2 minutes at 95°C; 25 cycles consisting of 98°C for 20 seconds, 60°C for 35 seconds, and 72°C for 3 minutes; and one cycle consisting of retention for 10 minutes at 72°C. This was followed by cooling at 4°C.

From the transformed calluses, RNA was extracted according to the method described in Tozawa et al., Plant Physiology 126: 1493-1506 (2001), and RNA blot hybridization was performed according to the method using a digoxigenin-labeled RNA probe for OASA2, as described in the same publication (Tozawa et al., Plant Physiology 126: 1493-1506 (2001)). RNA expression in the selected calluses was confirmed as the expression of OASA2 wild type gene or OASA2 mutant gene ((Y367A) or (Y367A/L530D)) that were introduced by transformation.

### <Analysis of Free Tryptophan Content in Transformed Calluses - 1>

One hundred mg of rice callus was crushed in liquid nitrogen for two lines of rice calluses: one expressing mutant OASA2 gene (Y367A, Y367A/L530D); and one expressing wild type (Wt) OASA2 gene. The crushed callus was transferred into a 1.5 ml tube, and a 500 µl mixture of chloroform, methanol, and water (volume ratio of 5:12:3) was added thereto. After thorough mixing, the mixture was centrifuged for 10 minutes at 5,000 × rpm. The supernatant was transferred into a new tube and supplemented with 375 µl of distilled water and 250 µl of chloroform. The mixture was vigorously agitated, and centrifuged at 5,000 × rpm for 10 minutes. The extracted aqueous layer was transferred into a new tube, and was evaporated under reduced pressure. This was dissolved in 2 ml of 10 mM NaOH to prepare a tryptophan extract. The preparation was then applied to a high-performance liquid chromatography (HPLC) device (the product of Waters, Waters Alliance HPLC FLD System 2695) and free tryptophan content was measured. For HPLC, a Xterra RP18 column (4.6 × 150 mm) (Waters) was used. The eluting solvent was used at the concentration gradient of acetonitrile-0.1M H₃PO₄ aqueous solution (pH 4.0), 5% to 45% of acetonitrile. At a flow rate of 1 ml/minute, tryptophan content was evaluated by the measurement at the excitation wavelength 278nm/fluorescence wavelength 348 nm.

As a control, calluses of non-transformed normal rice (variety: Nipponbare) were used. The results of measurement are shown in Table 3. As clearly shown in Table 3, the content of accumulated free tryptophan was greater in the rice calluses expressing the mutant OASA2 gene (Y367A, Y367A/L530D) having resistance to tryptophan feedback inhibition than in the control rice calluses (a 5.5 to 38.8 fold increase from wild type)

**[Table 3]**

| Measured Rice Callus | Free Tryptophan Content (nmol/g wet weight) | Relative Amount (fold) |
|---|---|---|
| Control Rice Callus (Nipponbare) | 32 ± 5 | 1.0 |
| pUB-OASA2(wt)-Hm-Introduced Callus: W22 | 7 ± 1 | 0.2 |
| pUB-OASA2(wt)-Hm-Introduced Callus: W28 | 20 ± 5 | 0.6 |
| pUB-OASA2(Y367A)-Hm-Introduced Callus: Y1 | 176 ± 12 | 5.5 |
| pUB-OASA2(Y367A)-Hm-Introduced Callus: Y29 | 532 ± 225 | 16.6 |
| pUB-OASA2(Y367A/L530D)-Hm-Introduced Callus: YL65 | 1,106 ± 311 1,106 ± 311 | 34.6 |
| pUB-OASA2(Y367A/L530D)-Hm-Introduced Callus: YL68 | 1,243 ± 184 | 38.8 |

### <Analysis of Free Tryptophan Content of Transformed Calluses - 2>

Evaluation of free tryptophan content was also made for 6 lines of rice calluses expressing mutant OASA2 gene (S126F/L530D), using the procedures described in the <Analysis of Free Tryptophan Content of Transformed Calluses - 1>.

As a control, non-transformed normal rice calluses (variety: Nipponbare) were used. The results of measurement are shown in Table 4. As clearly shown in Table 4, the content of accumulated free tryptophan was greater in the rice calluses expressing the mutant OASA2 gene (S126F/L530D) having resistance to tryptophan feedback inhibition than in the control rice calluses (a 123 to 467 fold increase from wild type)

**[Table 4]**

| Measured Rice Callus | Free Tryptophan Content (nmol/g wet weight) | Relative Amount (fold) |
|---|---|---|
| Control Rice Callus (Nipponbare) | 10 | 1 |
| pUB-OASA2(S126F/L530D)-Hm-Introduced Callus: SL7 | 1,196 | 123 |
| pUB-OASA2(S126F/L530D)-Hm-Introduced Callus: SL24 | 1,310 | 134 |
| pUB-OASA2(S126F/L530D)-Hm-Introduced Callus: SL38 | 3,005 | 308 |
| pUB-OASA2(S126F/L530D)-Hm-Introduced Callus: SL13 | 3,776 | 387 |
| pUB-OASA2(S126F/L530D)-Hm-Introduced Callus: SL27 | 3,927 | 403 |
| pUB-OASA2(S126F/L530D)-Hm-Introduced Callus: SL32 | 4,558 | 467 |

The embodiments and concrete examples of implementation discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

### INDUSTRIAL APPLICABILITY

The present invention realizes production of plants with a high tryptophan content. The invention is therefore has a wide range of agricultural applications. Further, since plants with a high tryptophan content are suitable for feedings or food materials, the present invention is also applicable to farming and food industry.

## Claims

1. A polypeptide which has a mutation at at least one of position 126, 367, and 369 of an amino acid sequence of SEQ ID NO: 1, wherein the polypeptide has resistance to tryptophan feedback inhibition in a biosynthetic pathway of tryptophan.

2. A polypeptide according to claim 1, which has a mutation at at least one of position 351, 522, and 530 of the amino acid sequence of SEQ ID NO: 1,
wherein the polypeptide has enzyme activity at least 0.7 times enzyme activity of wild type rice anthranilate synthase.

3. A polypeptide according to claim 1 or 2, wherein the mutation at position 126 of the amino acid sequence of SEQ ID NO: 1 is a substitution of phenylalanine for serine,
wherein the mutation at position 367 of the amino acid sequence of SEQ ID NO: 1 is a substitution of alanine or isoleucine for tyrosine, and
wherein the mutation at position 369 of the amino acid sequence of SEQ ID NO: 1 is a substitution of leucine for alanine.

4. A polypeptide according to any one of claims 1 through 3,
wherein the mutation at position 351 of the amino acid sequence of SEQ ID NO: 1 is a substitution of asparatate for asparagine,
wherein the mutation at position 522 of the amino acid sequence of SEQ ID NO: 1 is a substitution of tyrosine for glycin, and
wherein the mutation at position 530 of the amino acid sequence of SEQ ID NO: 1 is a substitution of alanine or asparatate for leucine.

5. A polypeptide according to any one of claims 1 through 4, which comprises:
an amino acid sequence of any one of SEQ ID NOs: 2 through 7 and SEQ ID NOs: 29 through 32; or
an amino acid sequence with a deletion, substitution, or addition of one or more amino acids in an amino acid sequence of any one of SEQ ID NOs: 2 through 7 and SEQ ID NOs: 29 through 32.

6. A polypeptide which has a mutation in a tryptophan binding region of rice anthranilate synthase, wherein the mutation occurs at position 5 of an amino acid sequence of SEQ ID NO: 26, and wherein the polypeptide has resistance to tryptophan feedback inhibition in a biosynthetic pathway of tryptophan.

7. A polypeptide according to claim 6, wherein the mutation at position 5 of the amino acid sequence of SEQ ID NO: 26 is a substitution of alanine or isoleucine for tyrosine.

8. A polynucleotide which encodes a polypeptide of any one of claims 1 through 7.

9. A polynucleotide according to claim 8, which has a base sequence of any one of SEQ ID NOs: 9 through 14 and SEQ ID NOs: 33 through 36, or a base sequence that hybridizes under stringent conditions with a base sequence complementary to the base sequence of any one of SEQ ID NOs: 9 through 14 and SEQ ID NOs: 33 through 36.

10. A marker gene for screening transformants, which comprises a polynucleotide of claim 8 or 9.

11. A recombinant expression vector which comprises a polynucleotide of claim 8 or 9.

12. A transformant which has incorporated therein a polynucleotide of claim 8 or 9 or a recombinant expression vector of claim 11, and in which a polypeptide having resistance to tryptophan feedback inhibition in a biosynthetic pathway of tryptophan is expressed.

13. A transformant according to claim 12, wherein the transformant is a plant cell or a plant.

14. A seed obtained from a plant of claim 13.

15. A method for screening transformed cells, comprising the steps of:
introducing into cells a marker gene of claim 10 or a recombinant expression vector of claim 11 so as to render the cells resistant to a tryptophan-like compound that inhibits proliferation of cells; and
screening for cells expressing resistance to the tryptophan-like compound.

16. A transformation kit comprising a polynucleotide of claim 8 or 9, or a recombinant expression vector of claim 11.

17. A method for screening for a substance that binds to at least one of a polypeptide of any one of claims 1 through 5 and a wild type rice anthranilate synthase,
the method comprising the steps of:
screening for a substance binding to a polypeptide of any one of claims 1 through 5;
screening for a substance binding to a wild type rice anthranilate synthase; and
comparing results of the screening steps.

18. A kit for performing a screening method of claim 17, which comprises a wild type rice anthranilate synthase and at least one of polypeptides of claims 1 through 5.
